(19) Europäisches Patentamt / European Patent Office / Office européen des brevets

(11) EP 4 534 668 A1

(12) EUROPEAN PATENT APPLICATION
published in accordance with Art. 153(4) EPC

(43) Date of publication:
**09.04.2025 Bulletin 2025/15**

(21) Application number: **23816054.3**

(22) Date of filing: **30.05.2023**

(51) International Patent Classification (IPC):
***C12N 15/11*** (2006.01) ***A61K 31/7088*** (2006.01)
***A61K 45/00*** (2006.01) ***A61K 48/00*** (2006.01)
***A61P 35/00*** (2006.01) ***A61P 37/04*** (2006.01)
***A61P 43/00*** (2006.01)

(52) Cooperative Patent Classification (CPC):
**A61K 31/7088; A61K 45/00; A61K 48/00;
A61P 35/00; A61P 37/04; A61P 43/00; C12N 15/11**

(86) International application number:
**PCT/JP2023/020104**

(87) International publication number:
**WO 2023/234297 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.05.2022 JP 2022088931**

(71) Applicant: **Kyoto University**
**Kyoto-shi, Kyoto 606-8501 (JP)**

(72) Inventors:
- **TAKEUCHI, Osamu**
  **Kyoto-shi, Kyoto 606-8501 (JP)**
- **UEHATA, Takuya**
  **Kyoto-shi, Kyoto 606-8501 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) COMPOSITION FOR TREATING CANCER

(57) The disclosure provides a composition for treating cancer comprising at least one substance that disrupts a stem-loop structure in the 3' untranslated region of an Nfkbiz mRNA, wherein the stem-loop structure is at least one stem-loop structure selected from a first stem-loop structure formed in a region corresponding to positions 34 to 50 of SEQ ID NO: 9, a second stem-loop structure formed in a region corresponding to positions 100 to 116 of SEQ ID NO: 9, and a third stem-loop structure formed in a region corresponding to positions 129 to 143 of SEQ ID NO: 9.

[Fig. 23]

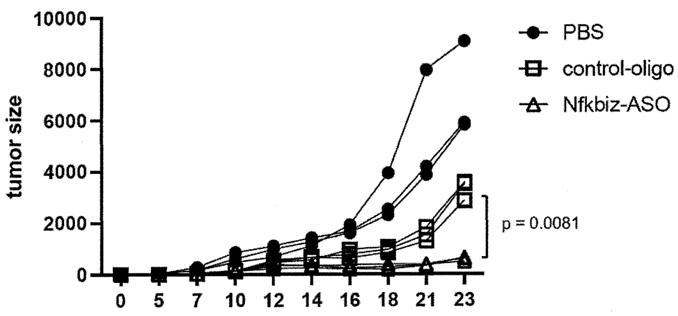




Processed by Luminess, 75001 PARIS (FR)

## Description

TECHNICAL FIELD

This application claims benefit of priority to Japanese Patent Application No. 2022-088931, the entire content of which is incorporated herein by reference.

The present disclosure relates to a composition for treating cancer.

BACKGROUND ART

Cancer immunotherapy has made rapid progress by virtue of the development of immune checkpoint inhibitors, but currently only about 30% of the patients can respond to the treatment. Cancer immune-cell therapy using chimeric antigen receptor (CAR) T cells has shown significant efficacy in some hematologic cancers and has been clinically applied, but its efficacy against solid tumors is insufficient and further improvement is required.

IκBζ is a transcription regulator that positively regulates immune responses. IκBζ belongs to the IκB family and is rapidly induced upon activation of TLR/IL-1R/IL-18R signaling. IκBζ binds to NF-κB subunit p50, enhances transcription by chromatin remodeling, and positively regulates natural killer cell (NK cell) function, Th17 differentiation, and antibody production by B cells. IκBζ is encoded by Nfkbiz and the regulation of Nfkbiz expression involves mRNA degradation.

Regnase-1 suppresses excessive immune responses by degrading mRNAs encoding molecules involved in inflammatory induction and proinflammatory cytokines, e.g., interleukin (IL)-6, in innate immune cells such as macrophages and dendritic cells. Regnase-1 also suppresses excessive T cell activation in the acquired immunity by degrading mRNAs encoding molecules involved in the T cell activation. Regnase-1 recognizes a stem-loop structure in the 3' untranslated region (3' UTR) of a mRNA and degrades the mRNA (Patent Document 1, Non-Patent Document 1). Non-Patent Document 1 discloses that the stem-loop structure in the 3' UTR of Nfkbiz mRNA is required for suppression of Nfkbiz expression by Regnase-1.

Regnase-3 is an RNase belonging to the same family as Regnase-1. Unlike Regnase-1, Regnase-3 is highly expressed in macrophages among immune cells, and is transcriptionally regulated by TLR and IFN signaling. Although the direct targets of Regnase-3 in macrophages are unknown, it has been demonstrated in vitro that Regnase-3 can bind to and degrade mRNAs including Regnase-1 mRNA. Regnase-3 is thus considered to be an essential RNase for immune homeostasis and an important regulator of the TLR/IFN pathway in macrophages (Non-Patent Document 2).

PRIOR ART DOCUMENT

PATENT DOCUMENT

Patent Document 1: WO 2019/182055

NON-PATENT DOCUMENT

Non-patent Document 1: Mino, T. et al., Cell 161, 1058-1073 (2015)
Non-patent Document 2: von Gamm M., et al., J Exp Med (2019) 216 (7): 1700-1723

SUMMARY OF THE INVENTION

PROBLEMS TO BE SOLVED BY THE INVENTION

An object of the disclosure is to provide a novel method of treating cancer.

SOLUTIONS TO THE PROBLEMS

The 3' UTR of Nfkbiz mRNA has five stem-loop structures conserved among species. The inventors have found that disruption of a specific stem-loop structure among them suppresses degradation of Nfkbiz mRNA by Regnase-1 and Regnase-3 to increase Nfkbiz expression and stimulates immune cells to enhance cytotoxic activity against cancer cells.

Accordingly, an aspect of the disclosure provides a composition for immunostimulation comprising at least one substance that disrupts a stem-loop structure in the 3' untranslated region of an Nfkbiz mRNA.

An aspect of the disclosure provides a composition for treating cancer comprising at least one substance that

disrupts a stem-loop structure in the 3' untranslated region of an Nfkbiz mRNA.

EFFECTS OF THE INVENTION

The disclosure allows immunostimulation in a subject. The disclosure also allows treating cancer by immunostimulation.

BRIEF DESCRIPTION OF THE DRAWINGS

[Fig. 1] Fig. 1 shows the genotypes of CreERT2+ mice used in the Examples and the experimental schedule.
[Fig. 2] Fig. 2 is a photograph of spleens isolated from the mice 14 days after gene deletions.
[Fig. 3] Fig. 3 shows the FACS plots of $CD62L^+CD44^-$ naive T cells and $CD62L^-CD44^+$ effector CD4-positive T cells in mouse splenocytes (upper panel), and FACS plots of CD69 expression in CD4-positive T cells (lower panel).
[Fig. 4] Fig. 4 shows the FACS plots of $CD62L^+CD44^-$ naive T cells, central memory T cells, and $CD62L^-CD44^+$ effector CD8-positive T cells in mouse splenocytes.
[Fig. 5] Fig. 5 shows the alignment of nucleotides at positions 29-148 of the 3' untranslated region of Nfkbiz gene among species and the regions capable of forming secondary structures (SL2-5).
[Fig. 6] Fig. 6 schematically shows the stem-loop structures (SL4 and 5) and disruption of the secondary structures by antisense nucleotides targeting the structures.
[Fig. 7] Fig. 7 shows the luciferase activities of HeLa cells transfected with a reporter construct containing the 3' UTR of mouse Nfkbiz gene and the antisense nucleic acids as shown in the figure. $**p < 0.01$, $***p < 0.001$.
[Fig. 8] Fig. 8 shows the luciferase activities of HeLa cells transfected with a reporter construct containing the 3' UTR of human Nfkbiz gene and the antisense nucleic acids as shown in the figure. $*p < 0.05$.
[Fig. 9] Fig. 9 schematically shows the Nfkbiz gene expression modified by antisense nucleic acids.
[Fig. 10] Fig. 10 schematically shows the anti-tumor effects of cytotoxic T cells.
[Fig. 11] Fig. 11 shows the workflow of an in vitro T cell experiment using OT-I CD8-positive T cells.
[Fig. 12] Fig. 12 shows the percentages (%) (upper panel) and mean fluorescence intensities (MFI) (lower panel) of CD8T cells producing IFN-y, granzyme-b, and TNF in the OT-I CD8-positive T cells transfected with the antisense nucleic acid. $****p < 0.0001$.
[Fig. 13] Fig. 13 shows the antitumor activity of OT-I CD8T cells transfected with the antisense nucleic acid against B16-OVA cell line. The vertical axis indicates the cytotoxicity and the horizontal axis indicates the ratio of the number of OT-I CD8T cells (Effector) to the number of B16-OVA cells (Target). $**p < 0.01$, $***p < 0.001$, $****p < 0.0001$.
[Fig. 14] Fig. 14 shows the changes in expression of Nfkbiz protein over time in mouse CD8-positive T cells transfected with the antisense nucleic acid.
[Fig. 15] Fig. 15 shows the changes in Nfkbiz expression over time in mouse bone marrow-derived macrophages transfected with the antisense nucleic acid. $**p < 0.01$, $***p < 0.001$, $****p < 0.0001$.
[Fig. 16] Fig. 16 shows the expression of Nfkbiz protein in mouse bone marrow-derived macrophages transfected with the antisense nucleic acid.
[Fig. 17] Fig. 17 shows the changes in Nfkbiz expression in the presence of Actinomycin D in mouse bone marrow-derived macrophages transfected with the antisense nucleic acid. $*p < 0.05$, $***p < 0.001$.
[Fig. 18] Fig. 18 shows the IL-6 levels in the culture supernatants of mouse bone marrow-derived macrophages transfected with the antisense nucleic acid. $***p < 0.001$, $****p < 0.0001$.
[Fig. 19] Fig. 19 schematically shows the antisense nucleic acids targeting stem-loop structures (SL4, SL5, or both SL4 and SL5) in the 3' UTR of Nfkbiz gene.
[Fig. 20] Fig. 20 shows the Nfkbiz expression levels in mouse bone marrow-derived macrophages transfected with the antisense nucleic acids. $*p < 0.05$, $***p < 0.001$.
[Fig. 21] Fig. 21 shows the expression of Nfkbiz protein in mouse bone marrow-derived macrophages transfected with the antisense nucleic acids.
[Fig. 22] Fig. 22 shows the workflow of an in vivo tumor model using B16 melanoma cell line.
[Fig. 23] Fig. 23 shows the changes in tumor size over time in the in vivo tumor model treated with mouse CD8-positive T cells transfected with the antisense nucleic acid.
[Fig. 24] Fig. 24 shows the FACS plots showing expression of PD-1, LAG-3, TOX, and TCF-1 in mouse CD8-positive T cells transfected with the antisense nucleic acid.
[Fig. 25] Fig. 25 shows the expression of NFKBIZ protein in human peripheral blood mononuclear cells (PBMCs) transfected with the antisense nucleic acid. Symbol * indicates nonspecific bands.

DETAILED DESCRIPTION

When a numerical value is accompanied with the term "about", the value is intended to represent any value in the range of -10% of the value to +10% of the value. For example, "about 20" means "a value from 18 to 22." A range defined with a value of the lower limit and a value of the upper limit covers all values from the lower limit to the upper limit, including the values of the limits. When a numerical range is accompanied with the term "about", both of the limits are read as accompanied with the term. For example, "about 20 to 30" is read as "18 to 33."

Unless otherwise defined, the terms used herein are read as generally understood by those skilled in the fields such as organic chemistry, medical sciences, pharmaceutical sciences, molecular biology, and microbiology. Several terms used herein are defined as below. The definitions herein take precedence over the general understanding.

Nfkbiz may be of any species, typically a mammal, e.g., human, mouse, rat, hamster, rabbit, cat, dog, cow, sheep, or monkey, particularly human or mouse. The representative amino acid sequences of IκBζ, which is encoded by Nfkbiz, are registered with GenBank accession numbers NP_001005474.1 (human, SEQ ID NO: 1) and NP_001152866.1 (mouse, SEQ ID NO: 2). The term "IκBζ" as used herein includes proteins having at least about 60%, about 70%, about 80%, about 85%, about 90%, about 95%, about 96%, about 97%, about 98%, or about 99% identity with the amino acid sequence of SEQ ID NO: 1 or 2, as long as they maintain the function of IκBζ.

The term "3' untranslated region of an Nfkbiz mRNA" or "3' UTR of an Nfkbiz mRNA" means the region at the 3' end of the Nfkbiz mRNA that is not translated. For example, the 3' UTR is the region from the nucleotide immediately following the stop codon of the mRNA to the 3' end. Nfkbiz mRNA as used herein may be any mRNA that comprises a nucleotide sequence encoding IκBζ as defined above and a nucleotide sequence of its 3' UTR. Examples of the nucleotide sequences of the 3' UTR of Nfkbiz mRNA are described below. The representative nucleotide sequences of IκB Nfkbiz mRNA are registered with GenBank accession numbers NM_001005474.3 (human, SEQ ID NO: 3) and NM_001159394.1 (mouse, SEQ ID NO: 4).

Examples of the nucleotide sequences of the 3' UTR of an Nfkbiz mRNA include sequences comprising at least the sequence of SEQ ID NO: 5 (human) or SEQ ID NO: 6 (mouse). For example, the 3' UTR of an Nfkbiz mRNA comprises the nucleotide sequence of SEQ ID NO: 7 (human) or SEQ ID NO: 8 (mouse). For example, the 3' UTR of an Nfkbiz mRNA has the nucleotide sequence of SEQ ID NO: 9 (human) or SEQ ID NO: 10 (mouse). In an embodiment, the 3' UTR of an Nfkbiz mRNA comprises a nucleotide sequence having at least about 60%, about 70%, about 80%, about 85%, about 90%, about 95%, about 96%, about 97%, about 98%, or about 99% identity with the nucleotide sequence of SEQ ID NO: 5 or 6. In an embodiment, the 3' UTR of an Nfkbiz mRNA comprises a nucleotide sequence having at least about 60%, about 70%, about 80%, about 85%, about 90%, about 95%, about 96%, about 97%, about 98%, or about 99% identity with the nucleotide sequence of SEQ ID NO: 7 or 8. In an embodiment, the 3' UTR of an Nfkbiz mRNA consists of a nucleotide sequence having at least about 60%, about 70%, about 80%, about 85%, about 90%, about 95%, about 96%, about 97%, about 98%, or about 99% identity with the nucleotide sequence of SEQ ID NO: 9 or 10.

In the disclosure the term "identity" of amino acid or nucleotide sequences means the degree of sequence similarity between two proteins or nucleic acids. The sequence identity is determined by optimally aligning two sequences to be compared, i.e., aligning the two sequences so that the number of matched amino acids or nucleotides is the largest, and identifying the amino acids or nucleotides matched in the two sequences. When the two sequences match exactly, the sequence identity is 100%. For example, the sequence identity can be calculated by the following formula.

$$\text{Sequence identity (\%)} = [(\text{number of identical residues})/(\text{alignment length})] \times 100$$

Algorithms for making the alignment and calculating the sequence identity may include any algorithm commonly available to those skilled in the art, e.g., BLAST algorithm or FASTA algorithm. The sequence identity may be determined using a software for sequence analysis such as BLAST or FASTA.

The term "stem-loop structure" means a structure in a single-stranded nucleic acid that is composed of a stem portion and a loop portion, in which the stem portion is formed with two complementary sequences located at two separate regions in the nucleic acid, and the loop portion is formed by the sequence located between the two regions. The 3' UTR of an Nfkbiz mRNA has five stem-loop structures conserved among species. For convenience of explanation, the five stem-loop structures are herein referred to as SL1, SL2, SL3, SL4 and SL5, from the 5' end of the 3' UTR of an Nfkbiz mRNA. In the disclosure, preferably at least one of SL2, SL4, and SL5 is disrupted. The nucleotide sequences of SEQ ID NOs: 5 and 6 above comprise SL4 and SL5. The nucleotide sequences of SEQ ID NOs: 7 and 8 above comprise SL2, SL3, SL4, and SL5. The nucleotide sequences of SEQ ID NOs: 9 and 10 above consist of the full length of the 3' UTR of each Nfkbiz mRNA and comprise all stem-loop structures (SL1, SL2, SL3, SL4, and SL5).

In an embodiment, in the 3' UTR of an Nfkbiz mRNA, a first stem-loop structure (SL2) formed in a region corresponding to positions 34 to 50 of SEQ ID NO: 9 (human), a second stem-loop structure (SL4) formed in a region corresponding to positions 100 to 116 of SEQ ID NO: 9, and/or a third stem-loop structure (SL5) formed in a region

corresponding to positions 129 to 143 of SEQ ID NO: 9 is disrupted. In an embodiment, in the 3' UTR of an Nfkbiz mRNA, a first stem-loop structure (SL2) formed in a region corresponding to positions 35 to 51 of SEQ ID NO: 10 (mouse), a second stem-loop structure (SL4) formed in a region corresponding to positions 101 to 117 of SEQ ID NO: 10, and/or a third stem-loop structure (SL5) formed in a region corresponding to positions 130 to 144 of SEQ ID NO: 10 is disrupted.

In an embodiment, at least one stem-loop structure selected from the second stem-loop structure and the third stem-loop structure is disrupted. In an embodiment, the second stem-loop structure and the third stem-loop structure are disrupted.

When a first nucleotide sequence and a second nucleotide sequence are optimally aligned, a region in the second sequence that is aligned along a given region in the first sequence is defined as the region corresponding to the given region in the first sequence.

The term "substance that disrupts a stem-loop structure" may be any substance that inhibits complementary binding within the stem-loop structure. Any substance that inhibits complementary binding of at least one, two, or three nucleotide pairs may be used. Examples of the substances include an oligonucleotide that binds to a nucleotide sequence forming a stem-loop structure (an antisense nucleic acid), or a substance that modifies a nucleotide sequence forming a stem-loop structure via genome editing.

In an embodiment, the substance that disrupts a stem-loop structure is an antisense nucleic acid. The antisense nucleic acid may inhibit complementary binding within a stem-loop structure by binding to at least one portion of the region forming the stem-loop structure in the 3' untranslated region of an Nfkbiz mRNA, e.g., at least one, two, or three nucleotides forming the stem portion of the stem-loop structure. For example, the antisense nucleic acid comprises the sequence complimentary to a sequence comprising at least two or three, e.g., three, contiguous nucleotides that form a stem portion of a stem-loop structure. The contiguous nucleotides may be adjacent to the loop portion of the stem-loop structure. For example, the contiguous nucleotides are at positions 35 to 37, 47 to 49, 104 to 106, 110 to 112, 132 to 134, and 138 to 140 of SEQ ID NO: 9, and positions 36 to 38, 48 to 50, 105 to 107, 111 to 113, 133 to 135, and 139 to 141 of SEQ ID NO: 10. Preferably, the antisense nucleic acid does not form a stem-loop structure, a hairpin structure, or a multimer such as a dimer, by itself. The antisense nucleic acid that disrupts one stem-loop structure consists of, for example, 10 to 30, 15 to 25, 18 to 22, or 19 to 21 nucleotides. The antisense nucleic acid that disrupts both of the second stem-loop structure and the third stem-loop structure consists of, for example, 25 to 35, 25 to 30, or 25 to 27 nucleotides.

In an embodiment, the antisense nucleic acid is selected from

(a-1) an oligonucleotide consisting of 10 to 30 nucleotides and being capable of binding to a sequence that consists of contiguous 10 to 30 nucleotides within positions 8 to 48 of SEQ ID NO: 9 and comprises the nucleotides at positions 35 to 37 of SEQ ID NO: 9, and

(a-2) an oligonucleotide consisting of 10 to 30 nucleotides and being capable of binding to a sequence that consists of contiguous 10 to 30 nucleotides within positions 36 to 76 of SEQ ID NO: 9 and comprises the nucleotides at positions 47 to 49 of SEQ ID NO: 9.

In an embodiment, the oligonucleotide (a-1) has at least about 60%, about 70%, about 80%, about 85%, about 90%, about 95%, about 96%, about 97%, about 98%, or about 99% sequence identity with SEQ ID NO: 11 (5'-taactg acagtgagtgttgc-3'). In another embodiment, the oligonucleotide (a-1) consists of a nucleotide sequence that is different from the nucleotide sequence of SEQ ID NO: 11 in that one or several, e.g., two or three, nucleotides are deleted, substituted, added, or inserted. In an embodiment, the oligonucleotide (a-1) is an oligonucleotide capable of binding to the nucleotide sequence complimentary to the nucleotide sequence of SEQ ID NO: 11. In an embodiment, the oligonucleotide (a-1) consists of a nucleotide sequence having at least about 90% identity with SEQ ID NO: 11. In an embodiment, the oligonucleotide (a-1) comprises the nucleotide sequence of SEQ ID NO: 11. In an embodiment, the oligonucleotide (a-1) consists of the nucleotide sequence of SEQ ID NO: 11.

In an embodiment, the oligonucleotide (a-2) has at least about 60%, about 70%, about 80%, about 85%, about 90%, about 95%, about 96%, about 97%, about 98%, or about 99% sequence identity with SEQ ID NO: 12 (5'-tacatc aggactgcctaact-3'). In another embodiment, the oligonucleotide (a-2) consists of a nucleotide sequence that is different from the nucleotide sequence of SEQ ID NO: 12 in that one or several, e.g., two or three, nucleotides are deleted, substituted, added, or inserted. In an embodiment, the oligonucleotide (a-2) is an oligonucleotide capable of binding to the nucleotide sequence complimentary to the nucleotide sequence of SEQ ID NO: 12. In an embodiment, the oligonucleotide (a-2) consists of a nucleotide sequence having at least about 90% identity with SEQ ID NO: 12. In an embodiment, the oligonucleotide (a-2) comprises the nucleotide sequence of SEQ ID NO: 12. In an embodiment, the oligonucleotide (a-2) consists of the nucleotide sequence of SEQ ID NO: 12.

In an embodiment, the antisense nucleic acid is selected from

(b-1) an oligonucleotide consisting of 10 to 30 nucleotides and being capable of binding to a sequence that consists of contiguous 10 to 30 nucleotides within positions 77 to 111 of SEQ ID NO: 9 and comprises the nucleotides at positions

104 to 106 of SEQ ID NO: 9, and

(b-2) an oligonucleotide consisting of 10 to 30 nucleotides and being capable of binding to a sequence that consists of contiguous 10 to 30 nucleotides within positions 105 to 139 of SEQ ID NO: 9 and comprises the nucleotides at positions 110 to 112 of SEQ ID NO: 9.

In an embodiment, the oligonucleotide (b-1) has at least about 60%, about 70%, about 80%, about 85%, about 90%, about 95%, about 96%, about 97%, about 98%, or about 99% sequence identity with SEQ ID NO: 13 (5'-atagaa acaacttacatttg-3'). In another embodiment, the oligonucleotide (b-1) consists of a nucleotide sequence that is different from the nucleotide sequence of SEQ ID NO: 13 in that one or several, e.g., two or three, nucleotides are deleted, substituted, added, or inserted. In an embodiment, the oligonucleotide (b-1) is an oligonucleotide capable of binding to the nucleotide sequence complimentary to the nucleotide sequence of SEQ ID NO: 13. In an embodiment, the oligonucleotide (b-1) consists of a nucleotide sequence having at least about 90% identity with SEQ ID NO: 13. In an embodiment, the oligonucleotide (b-1) comprises the nucleotide sequence of SEQ ID NO: 13. In an embodiment, the oligonucleotide (b-1) consists of the nucleotide sequence of SEQ ID NO: 13.

In an embodiment, the oligonucleotide (b-2) has at least about 60%, about 70%, about 80%, about 85%, about 90%, about 95%, about 96%, about 97%, about 98%, or about 99% sequence identity with SEQ ID NO: 14 (5'-ctaaat atgtttgtttcata-3'). In another embodiment, the oligonucleotide (b-2) consists of a nucleotide sequence that is different from the nucleotide sequence of SEQ ID NO: 14 in that one or several, e.g., two or three, nucleotides are deleted, substituted, added, or inserted. In an embodiment, the oligonucleotide (b-2) is an oligonucleotide capable of binding to the nucleotide sequence complimentary to the nucleotide sequence of SEQ ID NO: 14. In an embodiment, the oligonucleotide (b-2) consists of a nucleotide sequence having at least about 90% identity with SEQ ID NO: 14. In an embodiment, the oligonucleotide (b-2) comprises the nucleotide sequence of SEQ ID NO: 14. In an embodiment, the oligonucleotide (b-2) consists of the nucleotide sequence of SEQ ID NO: 14.

In an embodiment, the oligonucleotide (b-2) has at least about 60%, about 70%, about 80%, about 85%, about 90%, about 95%, about 96%, about 97%, about 98%, or about 99% sequence identity with SEQ ID NO: 38 (5'-aactaa atatgtttgtttc-3'). In another embodiment, the oligonucleotide (b-2) consists of a nucleotide sequence that is different from the nucleotide sequence of SEQ ID NO: 38 in that one or several, e.g., two or three, nucleotides are deleted, substituted, added, or inserted. In an embodiment, the oligonucleotide (b-2) is an oligonucleotide capable of binding to the nucleotide sequence complimentary to the nucleotide sequence of SEQ ID NO: 38. In an embodiment, the oligonucleotide (b-2) consists of a nucleotide sequence having at least about 90% identity with SEQ ID NO: 38. In an embodiment, the oligonucleotide (b-2) comprises the nucleotide sequence of SEQ ID NO: 38. In an embodiment, the oligonucleotide (b-2) consists of the nucleotide sequence of SEQ ID NO: 38.

In an embodiment, the antisense nucleic acid is selected from

(c-1) an oligonucleotide consisting of 10 to 30 nucleotides and being capable of binding to a sequence that consists of contiguous 10 to 30 nucleotides within positions 105 to 139 of SEQ ID NO: 9 and comprises the nucleotides at positions 132 to 134 of SEQ ID NO: 9, and

(c-2) an oligonucleotide consisting of 10 to 30 nucleotides and being capable of binding to a sequence that consists of contiguous 10 to 30 nucleotides within positions 133 to 167 of SEQ ID NO: 9 and comprises the nucleotides at positions 138 to 140 of SEQ ID NO: 9.

In an embodiment, the oligonucleotide (c-1) has at least about 60%, about 70%, about 80%, about 85%, about 90%, about 95%, about 96%, about 97%, about 98%, or about 99% sequence identity with SEQ ID NO: 15 (5'-ataata gtgaactaaatatg-3'). In another embodiment, the oligonucleotide (c-1) consists of a nucleotide sequence that is different from the nucleotide sequence of SEQ ID NO: 15 in that one or several, e.g., two or three, nucleotides are deleted, substituted, added, or inserted. In an embodiment, the oligonucleotide (c-1) is an oligonucleotide capable of binding to the nucleotide sequence complimentary to the nucleotide sequence of SEQ ID NO: 15. In an embodiment, the oligonucleotide (c-1) consists of a nucleotide sequence having at least about 90% identity with SEQ ID NO: 15. In an embodiment, the oligonucleotide (c-1) comprises the nucleotide sequence of SEQ ID NO: 15. In an embodiment, the oligonucleotide (c-1) consists of the nucleotide sequence of SEQ ID NO: 15.

In an embodiment, the oligonucleotide (c-1) has at least about 60%, about 70%, about 80%, about 85%, about 90%, about 95%, about 96%, about 97%, about 98%, or about 99% sequence identity with SEQ ID NO: 39 (5'-atagtg aactaaatatgt-3'). In another embodiment, the oligonucleotide (c-1) consists of a nucleotide sequence that is different from the nucleotide sequence of SEQ ID NO: 39 in that one or several, e.g., two or three, nucleotides are deleted, substituted, added, or inserted. In an embodiment, the oligonucleotide (c-1) is an oligonucleotide capable of binding to the nucleotide sequence complimentary to the nucleotide sequence of SEQ ID NO: 39. In an embodiment, the oligonucleotide (c-1) consists of a nucleotide sequence having at least about 90% identity with SEQ ID NO: 39. In an embodiment, the oligonucleotide (c-1) comprises the nucleotide sequence of SEQ ID NO: 39. In an embodiment, the oligonucleotide (c-1)

consists of the nucleotide sequence of SEQ ID NO: 39.

In an embodiment, the oligonucleotide (c-2) has at least about 60%, about 70%, about 80%, about 85%, about 90%, about 95%, about 96%, about 97%, about 98%, or about 99% sequence identity with SEQ ID NO: 16 (5'-ttaatat aacccactatata-3'). In another embodiment, the oligonucleotide (c-2) consists of a nucleotide sequence that is different from the nucleotide sequence of SEQ ID NO: 16 in that one or several, e.g., two or three, nucleotides are deleted, substituted, added, or inserted. In an embodiment, the oligonucleotide (c-2) is an oligonucleotide capable of binding to the nucleotide sequence complimentary to the nucleotide sequence of SEQ ID NO: 16. In an embodiment, the oligonucleotide (c-2) consists of a nucleotide sequence having at least about 90% identity with SEQ ID NO: 16. In an embodiment, the oligonucleotide (c-2) comprises the nucleotide sequence of SEQ ID NO: 16. In an embodiment, the oligonucleotide (c-2) consists of the nucleotide sequence of SEQ ID NO: 16.

In an embodiment, the antisense nucleic acid is
(d) an oligonucleotide consisting of 25 to 35 nucleotides and being capable of binding to a sequence that consists of contiguous 25 to 35 nucleotides within positions 105 to 139 of SEQ ID NO: 9 and comprises the nucleotides at positions 110 to 134 of SEQ ID NO: 9.

In an embodiment, the oligonucleotide (d) has at least about 60%, about 70%, about 80%, about 85%, about 90%, about 95%, about 96%, about 97%, about 98%, or about 99% sequence identity with SEQ ID NO: 17 (5'-atagtg aactaaatatgtttgtttc-3'). In another embodiment, the oligonucleotide (d) consists of a nucleotide sequence that is different from the nucleotide sequence of SEQ ID NO: 17 in that one or several, e.g., two or three, nucleotides are deleted, substituted, added, or inserted. In an embodiment, the oligonucleotide (d) is an oligonucleotide capable of binding to the nucleotide sequence complimentary to the nucleotide sequence of SEQ ID NO: 17. In an embodiment, the oligonucleotide (d) consists of a nucleotide sequence having at least about 90% identity with SEQ ID NO: 17. In an embodiment, the oligonucleotide (d) comprises the nucleotide sequence of SEQ ID NO: 17. In an embodiment, the oligonucleotide (d) consists of the nucleotide sequence of SEQ ID NO: 17.

One or more antisense nucleic acids may be used. When two or more antisense nucleic acids are used, a composition containing all antisense nucleic acids may be used, or two or more compositions each containing one or more antisense nucleic acids may be used in combination. When a composition contains two or more antisense nucleic acids or two or more antisense nucleic acids are administered simultaneously, the antisense nucleic acids preferably do not form a complementary bond between them.

In an embodiment, a combination of at least two antisense nucleic acids selected from the antisense nucleic acid (a-1) or (a-2), the antisense nucleic acid (b-1) or (b-2), and the antisense nucleic acid (c-1) or (c-2) is used. For example, the combination of the antisense nucleic acids (a-1)/(b-1), (a-1)/(b-2), (a-2)/(b-1), (a-2)/(b-2), (a-1)/(c-1), (a-1)/(c-2), (a-2)/(c-1), (a-2)/(c-2), (b-1)/(c-1), (b-1)/(c-2), (b-2)/(c-1), (b-2)/(c-2), (a-1)/(b-1)/(c-1), (a-1)/(b-1)/(c-2), (a-1)/(b-2)/(c-1), (a-1)/(b-2)/(c-2), (a-2)/(b-1)/(c-1), (a-2)/(b-1)/(c-2), (a-2)/(b-2)/(c-1), or (a-2)/(b-2)/(c-2) is used.

In an embodiment, a combination of the antisense nucleic acid (a-1) or (a-2) and the antisense nucleic acid (d) is used. For example, the combination of the antisense nucleic acids (a-1)/(d) or (a-2)/(d) is used.

In an embodiment, the antisense nucleic acid is selected from

(a-1') an oligonucleotide consisting of 10 to 30 nucleotides and being capable of binding to a sequence that consists of contiguous 10 to 30 nucleotides within positions 9 to 49 of SEQ ID NO: 10 and comprises the nucleotides at positions 36 to 38 of SEQ ID NO: 10, and

(a-2') an oligonucleotide consisting of 10 to 30 nucleotides and being capable of binding to a sequence that consists of contiguous 10 to 30 nucleotides within positions 37 to 77 of SEQ ID NO: 10 and comprises the nucleotides at positions 48 to 50 of SEQ ID NO: 10.

In an embodiment, the antisense nucleic acid is selected from

(b-1') an oligonucleotide consisting of 10 to 30 nucleotides and being capable of binding to a sequence that consists of contiguous 10 to 30 nucleotides within positions 78 to 112 of SEQ ID NO: 10 and comprises the nucleotides at positions 105 to 107 of SEQ ID NO: 10, and

(b-2') an oligonucleotide consisting of 10 to 30 nucleotides and being capable of binding to a sequence that consists of contiguous 10 to 30 nucleotides within positions 106 to 140 of SEQ ID NO: 10 and comprises the nucleotides at positions 111 to 113 of SEQ ID NO: 10.

In an embodiment, the antisense nucleic acid is selected from

(c-1') an oligonucleotide consisting of 10 to 30 nucleotides and being capable of binding to a sequence that consists of contiguous 10 to 30 nucleotides within positions 106 to 140 of SEQ ID NO: 10 and comprises the nucleotides at positions 133 to 135 of SEQ ID NO: 10, and

(c-2') an oligonucleotide consisting of 10 to 30 nucleotides and being capable of binding to a sequence that consists of contiguous 10 to 30 nucleotides within positions 134 to 168 of SEQ ID NO: 10 and comprises the nucleotides at positions 139 to 141 of SEQ ID NO: 10.

In an embodiment, the antisense nucleic acid is
(d') an oligonucleotide consisting of 25 to 35 nucleotides and being capable of binding to a sequence that consists of contiguous 25 to 35 nucleotides within positions 106 to 140 of SEQ ID NO: 10 and comprises the nucleotides at positions 111 to 135 of SEQ ID NO: 10.

The antisense nucleic acids (a-1'), (a-2'), (b-1'), (b-2'), (c-1'), (c-2'), and (d') are defined in accordance with the antisense nucleic acids (a-1), (a-2), (b-1), (b-2), (c-1), (c-2), and (d), respectively, as described above.

In an embodiment, the oligonucleotides (a-1'), (a-2'), (b-1'), (b-2'), (c-1'), (c-2'), and (d') have at least about 60%, about 70%, about 80%, about 85%, about 90%, about 95%, about 96%, about 97%, about 98%, or about 99% sequence identity with SEQ ID NO: 18 (5'-taactgacagtgagtgtcgc-3'), SEQ ID NO: 12, SEQ ID NO: 13, SEQ ID NO: 19 (5'-ctaaat gggtttgtttcata-3'), SEQ ID NO: 20 (5'-ataatagtgaactaaatggg-3'), SEQ ID NO: 21 (5'-ttaatataatccactatata-3'), and SEQ ID NO: 22 (5'-atagtgaactaaatgggtttgtttc-3'), respectively. In another embodiment, the oligonucleotides (a-1'), (a-2'), (b-1'), (b-2'), (c-1'), (c-2'), and (d') consist of a nucleotide sequence that is different from the nucleotide sequence of SEQ ID NOs: 18, 12, 13, 19, 20, 21, and 22, respectively, in that one or several, e.g., two or three, nucleotides are deleted, substituted, added, or inserted. In an embodiment, the oligonucleotides (a-1'), (a-2'), (b-1'), (b-2'), (c-1'), (c-2'), and (d') are an oligonucleotide capable of binding to the nucleotide sequence complimentary to the nucleotide sequence of SEQ ID NOs: 18, 12, 13, 19, 20, 21, and 22, respectively. In an embodiment, the oligonucleotides (a-1'), (a-2'), (b-1'), (b-2'), (c-1'), (c-2'), and (d') have a nucleotide sequence having at least about 90% identity with SEQ ID NOs: 18, 12, 13, 19, 20, 21, and 22, respectively. In an embodiment, the oligonucleotides (a-1'), (a-2'), (b-1'), (b-2'), (c-1'), (c-2'), and (d') comprise the nucleotide sequence of 18, 12, 13, 19, 20, 21, and 22, respectively. In an embodiment, the oligonucleotides (a-1'), (a-2'), (b-1'), (b-2'), (c-1'), (c-2'), and (d') consist of the nucleotide sequence of SEQ ID NOs: 18, 12, 13, 19, 20, 21, and 22, respectively.

In an embodiment, the oligonucleotides (b-2') and (c-1') have at least about 60%, about 70%, about 80%, about 85%, about 90%, about 95%, about 96%, about 97%, about 98%, or about 99% sequence identity with SEQ ID NO: 40 (5'-aactaaatgggtttgtttc-3') and SEQ ID NO: 41 (5'-atagtgaactaaatgggt-3'), respectively. In another embodiment, the oligonucleotides (b-2') and (c-1') consist of a nucleotide sequence that is different from the nucleotide sequence of SEQ ID NOs: 40 and 41, respectively, in that one or several, e.g., two or three, nucleotides are deleted, substituted, added, or inserted. In an embodiment, the oligonucleotides (b-2') and (c-1') are an oligonucleotide capable of binding to the nucleotide sequence complimentary to the nucleotide sequence of SEQ ID NOs: 40 and 41, respectively. In an embodiment, the oligonucleotides (b-2') and (c-1') have a nucleotide sequence having at least about 90% identity with SEQ ID NOs: 40 and 41, respectively. In an embodiment, the oligonucleotides (b-2') and (c-1') comprise the nucleotide sequence of 40 and 41, respectively. In an embodiment, the oligonucleotides (b-2') and (c-1') consist of the nucleotide sequence of SEQ ID NOs: 40 and 41, respectively.

The antisense nucleic acid may be composed of natural nucleotides, or composed of artificial nucleotides, or composed of one or more natural nucleotides and one or more artificial nucleotides. Examples of the natural nucleotides include deoxyribonucleotides and ribonucleotides. The artificial nucleotide may have a structure different from the natural nucleotides and increased nuclease resistance or binding affinity with the target sequence. For example, the artificial nucleotides described in Deleavey, G. F., & Damha, M. J. (2012). Designing chemically modified oligonucleotides for targeted gene silencing. Chemistry & biology, 19(8), 937-954, the entire contents of which are incorporated herein by reference, may be used. Examples of the artificial nucleotides include abasic nucleosides; arabinonucleosides, 2'-deoxyuridine, α-deoxyribonucleosides, β-L-deoxyribonucleosides, and nucleosides having any other sugar modification; peptide nucleic acids (PNAs), phosphonic ester nucleic acids (PHONAs), locked nucleic acids (LNAs), 2'-O,4'-C-ethylene-bridged nucleic acids (ENAs), constrained ethyl (cEt) nucleosides, and morpholino nucleic acids. Examples of the artificial nucleotides having sugar modifications include those having substituted pentoses such as 2'-o-methylribose, 2'-o-methoxyethylribose, 2'-deoxy-2'-fluororibose, or 3'-o-methylribose; 1',2'-deoxyribose; arabinose; substituted arabinoses; hexoses, and alpha-anomers. Examples of the artificial nucleotides having modified bases include those having pyrimidines such as 5-hydroxycytosine, 5-methylcytosine, 5-fluorouracil, or 4-thiouracil; purines such as 6-methyladenine or 6-thioguanosine; and other heterocyclic bases. The antisense nucleic acid may comprise artificial nucleotides of the same type or two or more different types.

In an embodiment, the antisense nucleic acid is a morpholino oligo. The morpholino oligo is an oligonucleotide that has a morpholine ring in place of the ribose and deoxyribose of RNA and DNA. For example, the morpholino oligo has a structure in which the following component is repeatedly linked.

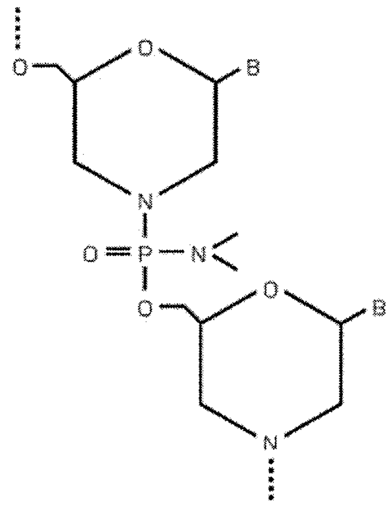

wherein B is a base, e.g., adenine, cytosine, guanine, thymine, or uracil, and the dashed line is the point connected with the adjacent component.

In an embodiment, the antisense nucleic acid is a single-stranded DNA.

The antisense nucleic acid may be bound to one or more components or conjugates that enhance the activity or cellular uptake of the antisense nucleic acid. Such components may include, but are not limited to, cholesterol components, cholic acid, thioethers (e.g., hexyl-S-tritylthiol), thiocholesterol, aliphatic chains (e.g., dodecanediol or undecyl residues), phospholipids (e.g., di-hexadecyl-rac-glycerol or triethylammonium 1,2-di-O-hexadecyl-rac-glycero-3-H-phosphonate), polyamine or polyethylene glycol chains, or adamantane acetic acid, palmitic components, or octadecylamine, hexylamino-carbonyl-t-oxycoresterol, or octaguanidine dendrimer components. Oligonucleotides containing such components and methods of preparing such oligonucleotides are known in the art.

The composition disclosed herein can stimulate immunity and thus can be used for treating cancer by cancer immunotherapy. In the disclosure, cancer may be any cancer, and may be a primary cancer or a metastatic cancer. Examples of the cancers include malignant melanoma, non-small cell lung cancer, small cell lung cancer, renal cell carcinoma, classical Hodgkin lymphoma, head and neck cancer, gastric cancer, malignant pleural mesothelioma, colorectal cancer, esophageal cancer, cancer of unknown primary, urothelial carcinoma, hepatocellular carcinoma, solid tumor, breast cancer, endometrial cancer, malignant pleural mesothelioma, Merkel cell carcinoma, brain tumor, bile duct cancer (including intrahepatic bile duct cancer), renal cancer, and colon cancer. In an embodiment, the cancer is malignant melanoma, non-small cell lung cancer, small cell lung cancer, renal cell carcinoma, classical Hodgkin lymphoma, head and neck cancer, gastric cancer, malignant pleural mesothelioma, colorectal cancer, esophageal cancer, cancer of unknown primary, urothelial carcinoma, hepatocellular carcinoma, solid tumor, breast cancer, endometrial cancer, malignant pleural mesothelioma, or Merkel cell carcinoma. In an embodiment, the cancer is brain tumor or bile duct cancer. In an embodiment, the cancer is malignant melanoma, renal cancer, or colon cancer. In an embodiment, the cancer is malignant melanoma.

The term "treating" or "treatment" as used herein means reducing or eliminating cancer cells; delaying or stopping cancer progression, recurrence, or metastasis; and/or reducing, alleviating, ameliorating, or removing cancer symptoms in a cancer patient.

The subjects of the treatment or prevention include animals, typically mammals (e.g., humans, mice, rats, hamsters, rabbits, cats, dogs, cows, sheep, or monkeys), especially humans.

Cancer immunotherapy may be immune cell therapy, such as CAR-T cell therapy. Immune cells may be treated with the composition disclosed herein. Immune cells may be any cells involved in immune response, including lymphoid cells such as T cells, NK cells, and B cells; antigen-presenting cells such as monocytes, macrophages, and dendritic cells; and granulocytes such as neutrophils, eosinophils, basophils, and mast cells. Isolated or purified immune cells or cell populations containing immune cells (e.g., peripheral blood mononuclear cells, cord blood cells) may be treated with the composition disclosed herein. T cells may be obtained by isolating or purifying the T cells from body fluids such as blood and bone marrow fluid, tissues such as spleen, thymus, and lymph nodes, or immune cells infiltrating cancerous tissues such as primary tumors, metastatic tumors, and cancerous ascites. T cells may be produced from pluripotent stem cells such as ES cells and iPS cells. Examples of the T cells include alpha-beta T cells, gamma-delta T cells, CD8-positive T cells, CD4-positive T cells, tumor-infiltrating T cells, memory T cells, naive T cells, NKT cells, and naive CD8-positive T cells. Immune cells may be isolated from the subject.

The composition disclosed herein is used to introduce the substance that disrupts a stem-loop structure into immune cells. The method of introduction is not limited, and methods normally used in immune cell therapies such as CAR-T cell therapy may be employed. For example, nucleic acids can be introduced by known methods such as calcium phosphate method, lipofection, microinjection, and electroporation. Thus, the composition may comprise a substance that disrupts a stem-loop structure in the 3' UTR of an Nfkbiz mRNA together with a substance to assist transfection. Alternatively, the composition may be a solution or lyophilized product comprising the substance that disrupts a stem-loop structure.

Immune cells treated with the composition disclosed herein may be administered to a subject. The method of administration is not limited and may be a method commonly used in immune cell therapy. For example, the cells may be administered in carotid artery, subcutaneously, intradermally, intratumorally, intralymphatically, intraspinally, intramuscularly, intravenously, or intraperitoneally. A composition comprising the immune cells may also be administered.

In cancer immunotherapy the composition disclosed herein may be administered to a subject. The administration routes of the composition include oral or parenteral routes and are not particularly limited. Various known dosage forms may be employed with the application site and the treated disease taken into consideration. For example, the parenteral administration may be systemic or local administration, more specifically, intratracheal, intraspinal, intrathecal, intracranial, intravenous, intraarterial, intraportal, intradermal, subcutaneous, intramuscular, intraperitoneal, intranasal, or intraoral administration. In an embodiment, the composition is administered intravenously.

Dosage forms such as granules, fine granules, powders, coated tablets, tablets, suppositories, fine powders, capsules, microcapsules, chewable tablets, liquids, suspensions, and emulsions may be employed. Dosage forms that prolong the release of the active ingredient may be employed. Dosage forms for injection or infusion include aqueous and non-aqueous injectable solutions, which may comprise an excipient, such as an antioxidant, a buffer, a bacteriostatic agent, or an isotonic agent; and aqueous and non-aqueous injectable suspensions, which may comprise an excipient, such as a suspending agent or thickening agent. Such dosage forms may be provided as liquids in sealed ampoules or vials, or provided as lyophilized products and prepared immediately prior to use by adding sterile liquids such as water for injection. The injectable solutions or suspensions may be prepared from powders, granules, or tablets.

Such dosage forms can be manufactured by formulating an active ingredient by conventional methods. If necessary for the formulation, any one of various pharmaceutically acceptable excipients may be added. Any excipient may be used in accordance with the employed dosage form. Examples of the excipients include buffering agents, surfactants, stabilizers, preservatives, fillers, diluents, additives, disintegrants, binders, coating agents, lubricants, lubricating agents, flavoring agents, sweeteners, and solubilizers.

The dosage and the number of doses of the composition may be appropriately set by those skilled in the art so that an effective amount of the active ingredient is administered to the subject, on the basis of factors such as the animal species, health condition, age, and weight of the subject, the administration route, and the employed dosage form. Those skilled in the art may easily determine the effective amount in a given situation by routine experimentation, which is within the range of ordinary skill and determination of clinicians. For example, when the active ingredient is an antisense nucleic acid, it may be administered in the range of about 0.01 to 100 mg/kg body weight, about 0.05 to 10 mg/kg body weight, or about 0.1 to 5 mg/kg body weight.

The composition may be used alone or in combination with at least one further active ingredient, especially an active ingredient for treating cancer. When some ingredients are used in combination, a dosage form containing all the ingredients or a combination of dosage forms containing each ingredient separately may be employed. The ingredients may be simultaneously or sequentially administered or any ingredient may be administered at later time point, as long as the ingredients are used for treating cancer. Two or more further active ingredients may be used in combination. Examples of the active ingredients suitable for use in combination include known anticancer agents.

Examples of the known anticancer agents include immune checkpoint inhibitors, alkylating agents, antimetabolites, anticancer antibiotics, plant alkaloids, antihormonal agents, platinum compounds, cytokine agents, molecular targeting agents, tumor immunotherapeutics, and cancer vaccines. In an embodiment, an immune checkpoint inhibitor is used in combination with the composition. Immune checkpoint inhibitors are substances that inhibit the function of immune checkpoint molecules, which suppress autoimmune responses and/or excessive immune responses, including, e.g., CTLA-4, PD-1, PD-L1, PD-L2, LAG-3, TIM3, BTLA, B7H3, B7H4, 2B4, CD160, A2aR, KIR, VISTA, and TIGIT.

Another cancer therapy may be performed in combination with cancer immunotherapy. Suitable therapies include, for example, chemotherapy, radiation therapy, hematopoietic stem cell transplantation, endoscopic resection, and surgery.

It is known that IL-18R is expressed in tumor-infiltrating CD8T cells but its signal is not transduced due to decoys derived from cancer cells. Without limited by theory, for example, because IκB-ζ acts downstream of IL-18R signaling, the composition disclosed herein is considered to activate T cells independently of binding of IL-18 and IL-18R and be able to activate exhausted T cells that have lost IL-18R expression. The composition is also expected to boost T cells resistant to PD-1 therapy, and to inhibit differentiation of tumor-infiltrating T lymphocytes into exhausted T cells and promote differentiation into effector T cells. Anti-tumor effects by targeting NK cells, which exert anti-tumor immunity through IL-18R signaling, are also expected. Increased Nfkbiz expression can also activate immune responses of macrophages and dendritic cells, and is expected to enhance cancer immunity.

An aspect of the disclosure provides a method of treating cancer comprising administering an effective amount of at least one substance that disrupts a stem-loop structure in the 3' untranslated region of an Nfkbiz mRNA to a subject in need thereof.

An aspect of the disclosure provides at least one substance that disrupts a stem-loop structure in the 3' untranslated region of an Nfkbiz mRNA for use in treating cancer.

An aspect of the disclosure provides use of at least one substance that disrupts a stem-loop structure in the 3' untranslated region of an Nfkbiz mRNA for treating cancer.

An aspect of the disclosure provides use of at least one substance that disrupts a stem-loop structure in the 3' untranslated region of an Nfkbiz mRNA for manufacturing a composition for treating cancer.

Another aspect of the disclosure provides a method of screening for an agent for treating cancer comprising

(a) introducing a candidate substance to a cell expressing Regnase-1 and/or Regnase-3 and a reporter gene fused with the 3' untranslated region of an Nfkbiz mRNA;
(b) measuring the expression level of the reporter gene in the cell, and
(c) identifying the candidate substance as the agent for treating cancer when the expression level measured in the presence of the candidate substance is higher than the expression level measured in the absence of the candidate substance.

In step (a) of the method, the 3' untranslated region of an Nfkbiz mRNA is as described above, for example, comprises at least the nucleotide sequence of SEQ ID NO: 5 or 6. The reporter gene may be any reporter gene that directly or indirectly generates a detectable label, such as chloramphenicol acetyltransferase (CAT) gene, green fluorescent protein (GFP) gene, β-glucuronidase (GUS) gene, luciferase gene, and other marker genes. The 3' untranslated region of an Nfkbiz mRNA is linked downstream of the reporter gene.

Regnase-1 and Regnase-3 may be of any species, typically a mammal, e.g., human, mouse, rat, hamster, rabbit, cat, dog, cow, sheep, or monkey, particularly human or mouse. The representative amino acid sequences of Regnase-1 are registered with GenBank accession numbers NP_001310479.1 (human, SEQ ID NO: 23) and NP_694799.1 (mouse, SEQ ID NO: 24). The representative nucleotide sequences of Regnase-1 are registered with GenBank accession numbers NM_001323550 (human, SEQ ID NO: 25) and NM_153159.2 (mouse, SEQ ID NO: 26). The representative amino acid sequences of Regnase-3 are registered with GenBank accession numbers NP_203748.1 (human, SEQ ID NO: 27) and NP_001156393.1 (mouse, SEQ ID NO: 28). The representative nucleotide sequences of Regnase-3 are registered with GenBank accession numbers NM_033390.2 (human, SEQ ID NO: 29) and NM_001162921.2 (mouse, SEQ ID NO: 30).

The terms "Regnase-1" and "Regnase-3" as used herein include proteins having at least about 60%, about 70%, about 80%, about 85%, about 90%, about 95%, about 96%, about 97%, about 98%, or about 99% identity with the amino acid sequences of SEQ ID NO: 23 or 24 and SEQ ID NO: 27 or 28, respectively, as long as they maintain the function of Regnase-1 and Regnase-3, respectively. The Regnase-1 and Regnase-3 may be intrinsically expressed by the cell used in the method or may be expressed by a gene introduced into the cell by a genetic recombination technique.

The cell used in the method may be any cell that is capable of expressing the reporter gene linked to the 3' untranslated region of an Nfkbiz mRNA and, if necessary, Regnase-1 and/or Regnase-3 gene. Examples of the cells include HeLa cells, HEK293 cells, HepG2 cells, and COS-7 cells. Other necessary steps such as construction of a transfection vector and transfection may be performed by known methods (Molecular Cloning: A Laboratory Manual 2nd edition (1989), Cold Spring Harbor Laboratory Press).

The candidate substance may be any substance, for example, a protein, an amino acid, a nucleic acid, a lipid, a carbohydrate, and a small molecule. The candidate substance is typically a purified or isolated substance, but may be provided in an unpurified or unisolated crude material. The candidate substance may be provided in a library, such as a compound library, a nucleic acid library, or a random peptide library, or may be provided in a natural material. The candidate substance may be designed based on a stem-loop structure in the 3' untranslated region of an Nfkbiz mRNA. The introduction of the candidate substance into the cell may be performed by a known method, depending on the type of the candidate substance.

In Step (b) of the method, the expression level of the reporter gene may be measured by a known method suitable for the reporter gene.

In Step (c) of the method, the candidate substance may be identified as the agent for treating cancer when the expression level of the reporter gene measured in the presence of the candidate substance is, e.g., at least about 10%, preferably at least about 20%, more preferably at least about 30%, even more preferably at least about 50%, higher than the expression level measured in the absence of the candidate substance.

An aspect of the disclosure provides a composition for immunostimulation comprising at least one substance that disrupts a stem-loop structure in the 3' untranslated region of an Nfkbiz mRNA. The term "immunostimulation" as used herein means to activate immune cells, to enhance immunity, and/or to remove immunosuppression. The immunity may

be innate immunity or acquired immunity. Details of the substance that disrupts a stem-loop structure and the method of use of the composition are as those for the composition for treating cancer described above.

An aspect of the disclosure provides a method of immunostimulation comprising administering an effective amount of at least one substance that disrupts a stem-loop structure in the 3' untranslated region of an Nfkbiz mRNA to a subject in need thereof.

An aspect of the disclosure provides at least one substance that disrupts a stem-loop structure in the 3' untranslated region of an Nfkbiz mRNA for use in immunostimulation.

An aspect of the disclosure provides use of at least one substance that disrupts a stem-loop structure in the 3' untranslated region of an Nfkbiz mRNA for immunostimulation.

An aspect of the disclosure provides use of at least one substance that disrupts a stem-loop structure in the 3' untranslated region of an Nfkbiz mRNA for manufacturing a composition for immunostimulation.

An aspect of the disclosure provides a substance that disrupts a stem-loop structure in the 3' untranslated region of an Nfkbiz mRNA. Details of the substance is as described above. Two or more substances may be used in combination.

An aspect of the disclosure provides a method of stimulating an immune cell comprising introducing in vitro at least one substance that disrupts a stem-loop structure in the 3' untranslated region of an Nfkbiz mRNA to the immune cell. The method may comprise a step of isolating the immune cell from a subject.

An aspect of the disclosure provides an immune cell comprising at least one substance that disrupts a stem-loop structure in the 3' untranslated region of an Nfkbiz mRNA.

An aspect of the disclosure provides a composition for treating cancer comprising an immune cell comprising at least one substance that disrupts a stem-loop structure in the 3' untranslated region of an Nfkbiz mRNA.

In such aspects the immune cell is as described above, for example, a naive CD8-positive T cell.

For example, the disclosure provides the following embodiments.

[1] A composition for immunostimulation, comprising at least one substance that disrupts a stem-loop structure in the 3' untranslated region of an Nfkbiz mRNA.

[2] A composition for treating cancer, comprising at least one substance that disrupts a stem-loop structure in the 3' untranslated region of an Nfkbiz mRNA.

[3] The composition according to item 1 or 2, wherein the stem-loop structure is at least one stem-loop structure selected from the following stem-loop structures;

a first stem-loop structure formed in a region corresponding to positions 34 to 50 of SEQ ID NO: 9,
a second stem-loop structure formed in a region corresponding to positions 100 to 116 of SEQ ID NO: 9, and
a third stem-loop structure formed in a region corresponding to positions 129 to 143 of SEQ ID NO: 9.

[4] The composition according to any one of items 1 to 3, wherein the composition comprises at least one substance that disrupts at least one stem-loop structure selected from the second stem-loop structure and the third stem-loop structure.

[5] The composition according to any one of items 1 to 4, wherein the composition comprises at least one substance that disrupts the second stem-loop structure and the third stem-loop structure.

[6] The composition according to any one of items 1 to 5, wherein the 3' untranslated region of the Nfkbiz mRNA comprises a nucleotide sequence having at least about 90% identity with the nucleotide sequence of SEQ ID NO: 5.

[7] The composition according to any one of items 1 to 6, wherein the 3' untranslated region of the Nfkbiz mRNA comprises the nucleotide sequence of SEQ ID NO: 5.

[8] The composition according to any one of items 1 to 7, wherein the 3' untranslated region of the Nfkbiz mRNA comprises a nucleotide sequence having at least about 90% identity with the nucleotide sequence of SEQ ID NO: 7.

[9] The composition according to any one of items 1 to 8, wherein the 3' untranslated region of the Nfkbiz mRNA comprises the nucleotide sequence of SEQ ID NO: 7.

[10] The composition according to any one of items 1 to 9, wherein the 3' untranslated region of the Nfkbiz mRNA consists of a nucleotide sequence having at least about 90% identity with the nucleotide sequence of SEQ ID NO: 9.

[11] The composition according to any one of items 1 to 10, wherein the 3' untranslated region of the Nfkbiz mRNA consists of the nucleotide sequence of SEQ ID NO: 9.

[12] The composition according to any one of items 1 to 11, wherein the at least one substance that disrupts a stem-loop structure is an oligonucleotide that binds to a nucleotide sequence forming the stem-loop structure.

[13] The composition according to any one of items 1 to 12, wherein the at least one substance that disrupts the stem-loop structure is

(a) a first oligonucleotide selected from

(a-1) an oligonucleotide consisting of 10 to 30 nucleotides and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of 10 to 30 nucleotides and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of 10 to 30 nucleotides and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of 10 to 30 nucleotides and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of 10 to 30 nucleotides and being capable of binding to a sequence that consists of contiguous 10 to 30 nucleotides within positions 105 to 139 of SEQ ID NO: 9 and comprises the nucleotides at positions 132 to 134 of SEQ ID NO: 9, and
(c-2) an oligonucleotide consisting of 10 to 30 nucleotides and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of 25 to 35 nucleotides and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

[14] The composition according to any one of items 1 to 13, wherein the at least one substance that disrupts the stem-loop structure is

(a) a first oligonucleotide selected from

(a-1) an oligonucleotide consisting of a nucleotide sequence having at least 90% identity with SEQ ID NO: 11 and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of a nucleotide sequence having at least 90% identity with SEQ ID NO: 12 and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of a nucleotide sequence having at least 90% identity with SEQ ID NO: 13 and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of a nucleotide sequence having at least 90% identity with SEQ ID NO: 14 and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of a nucleotide sequence having at least 90% identity with SEQ ID NO: 15 and being capable of binding to a sequence that consists of contiguous 10 to 30 nucleotides within positions

105 to 139 of SEQ ID NO: 9 and comprises the nucleotides at positions 132 to 134 of SEQ ID NO: 9, and
(c-2) an oligonucleotide consisting of a nucleotide sequence having at least 90% identity with SEQ ID NO: 16 and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of a nucleotide sequence having at least 90% identity with SEQ ID NO: 17 and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

[15] The composition according to any one of items 1 to 14, wherein the at least one substance that disrupts the stem-loop structure is

(a) a first oligonucleotide selected from

(a-1) an oligonucleotide consisting of a nucleotide sequence that is different from the nucleotide sequence of SEQ ID NO: 11 in that one, two or three nucleotides are deleted, substituted, added, or inserted, and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of a nucleotide sequence that is different from the nucleotide sequence of SEQ ID NO: 12 in that one, two or three nucleotides are deleted, substituted, added, or inserted, and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of a nucleotide sequence that is different from the nucleotide sequence of SEQ ID NO: 13 in that one, two or three nucleotides are deleted, substituted, added, or inserted, and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of a nucleotide sequence that is different from the nucleotide sequence of SEQ ID NO: 14 in that one, two or three nucleotides are deleted, substituted, added, or inserted, and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of a nucleotide sequence that is different from the nucleotide sequence of SEQ ID NO: 15 in that one, two or three nucleotides are deleted, substituted, added, or inserted, and being capable of binding to a sequence that consists of contiguous 10 to 30 nucleotides within positions 105 to 139 of SEQ ID NO: 9 and comprises the nucleotides at positions 132 to 134 of SEQ ID NO: 9, and
(c-2) an oligonucleotide consisting of a nucleotide sequence that is different from the nucleotide sequence of SEQ ID NO: 16 in that one, two or three nucleotides are deleted, substituted, added, or inserted, and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of a nucleotide sequence that is different from the nucleotide sequence of SEQ ID NO: 17 in that one, two or three nucleotides are deleted, substituted, added, or inserted, and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

[16] The composition according to any one of items 1 to 13, wherein the at least one substance that disrupts the stem-loop structure is

(a) a first oligonucleotide selected from

(a-1) an oligonucleotide capable of binding to the nucleotide sequence complimentary to the nucleotide sequence of SEQ ID NO: 11, and
(a-2) an oligonucleotide capable of binding to the nucleotide sequence complimentary to the nucleotide sequence of SEQ ID NO: 12,

(b) a second oligonucleotide selected from

(b-1) an oligonucleotide capable of binding to the nucleotide sequence complimentary to the nucleotide sequence of SEQ ID NO: 13, and
(b-2) an oligonucleotide capable of binding to the nucleotide sequence complimentary to the nucleotide sequence of SEQ ID NO: 14,

(c) a third oligonucleotide selected from

(c-1) an oligonucleotide capable of binding to the nucleotide sequence complimentary to the nucleotide sequence of SEQ ID NO: 15, and
(c-2) an oligonucleotide capable of binding to the nucleotide sequence complimentary to the nucleotide sequence of SEQ ID NO: 16,

(d) a fourth oligonucleotide capable of binding to the nucleotide sequence complimentary to the nucleotide sequence of SEQ ID NO: 17,
(e) a combination of at least two oligonucleotides selected from the first oligonucleotide, the second oligonucleotide, and the third oligonucleotide,
or
(f) a combination of the first oligonucleotide and the fourth oligonucleotide.

[17] The composition according to any one of items 1 to 16, wherein the at least one substance that disrupts the stem-loop structure is

(a) a first oligonucleotide selected from
(b) a second oligonucleotide selected from

(a-1) an oligonucleotide comprising the nucleotide sequence of SEQ ID NO: 11, and
(a-2) an oligonucleotide comprising the nucleotide sequence of SEQ ID NO: 12, (b) a second oligonucleotide selected from
(b-1) an oligonucleotide comprising the nucleotide sequence of SEQ ID NO: 13, and
(b-2) an oligonucleotide comprising the nucleotide sequence of SEQ ID NO: 14,

(c) a third oligonucleotide selected from

(c-1) an oligonucleotide comprising the nucleotide sequence of SEQ ID NO: 15, and
(c-2) an oligonucleotide comprising the nucleotide sequence of SEQ ID NO: 16,

(d) a fourth oligonucleotide comprising the nucleotide sequence of SEQ ID NO: 17,
(e) a combination of at least two oligonucleotides selected from the first oligonucleotide, the second oligonucleotide, and the third oligonucleotide,
or
(f) a combination of the first oligonucleotide and the fourth oligonucleotide.

[18] The composition according to any one of items 1 to 17, wherein the at least one substance that disrupts the stem-loop structure is

(a) a first oligonucleotide selected from

(a-1) an oligonucleotide consisting of the nucleotide sequence of SEQ ID NO: 11, and
(a-2) an oligonucleotide consisting of the nucleotide sequence of SEQ ID NO: 12,

(b) a second oligonucleotide selected from

(b-1) an oligonucleotide consisting of the nucleotide sequence of SEQ ID NO: 13, and
(b-2) an oligonucleotide consisting of the nucleotide sequence of SEQ ID NO: 14,

(c) a third oligonucleotide selected from

(c-1) an oligonucleotide consisting of the nucleotide sequence of SEQ ID NO: 15, and
(c-2) an oligonucleotide consisting of the nucleotide sequence of SEQ ID NO: 16,

(d) a fourth oligonucleotide consisting of the nucleotide sequence of SEQ ID NO: 17,
(e) a combination of at least two oligonucleotides selected from the first oligonucleotide, the second oligonucleotide, and the third oligonucleotide,
or
(f) a combination of the first oligonucleotide and the fourth oligonucleotide.

[19] The composition according to any one of items 1 to 13, wherein the at least one substance that disrupts the stem-loop structure is

(a) a first oligonucleotide selected from

(a-1) an oligonucleotide consisting of a nucleotide sequence having at least 90% identity with SEQ ID NO: 11 and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of a nucleotide sequence having at least 90% identity with SEQ ID NO: 12 and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of a nucleotide sequence having at least 90% identity with SEQ ID NO: 13 and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of a nucleotide sequence having at least 90% identity with SEQ ID NO: 38 and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of a nucleotide sequence having at least 90% identity with SEQ ID NO: 39 and being capable of binding to a sequence that consists of contiguous 10 to 30 nucleotides within positions 105 to 139 of SEQ ID NO: 9 and comprises the nucleotides at positions 132 to 134 of SEQ ID NO: 9, and
(c-2) an oligonucleotide consisting of a nucleotide sequence having at least 90% identity with SEQ ID NO: 16 and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of a nucleotide sequence having at least 90% identity with SEQ ID NO: 17 and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

[20] The composition according to any one of items 1 to 13 and 19, wherein the at least one substance that disrupts the

stem-loop structure is

(a) a first oligonucleotide selected from

(a-1) an oligonucleotide consisting of a nucleotide sequence that is different from the nucleotide sequence of SEQ ID NO: 11 in that one, two or three nucleotides are deleted, substituted, added, or inserted, and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of a nucleotide sequence that is different from the nucleotide sequence of SEQ ID NO: 12 in that one, two or three nucleotides are deleted, substituted, added, or inserted, and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of a nucleotide sequence that is different from the nucleotide sequence of SEQ ID NO: 13 in that one, two or three nucleotides are deleted, substituted, added, or inserted, and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of a nucleotide sequence that is different from the nucleotide sequence of SEQ ID NO: 38 in that one, two or three nucleotides are deleted, substituted, added, or inserted, and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of a nucleotide sequence that is different from the nucleotide sequence of SEQ ID NO: 39 in that one, two or three nucleotides are deleted, substituted, added, or inserted, and being capable of binding to a sequence that consists of contiguous 10 to 30 nucleotides within positions 105 to 139 of SEQ ID NO: 9 and comprises the nucleotides at positions 132 to 134 of SEQ ID NO: 9, and
(c-2) an oligonucleotide consisting of a nucleotide sequence that is different from the nucleotide sequence of SEQ ID NO: 16 in that one, two or three nucleotides are deleted, substituted, added, or inserted, and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of a nucleotide sequence that is different from the nucleotide sequence of SEQ ID NO: 17 in that one, two or three nucleotides are deleted, substituted, added, or inserted, and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

[21] The composition according to any one of items 1 to 13, wherein the at least one substance that disrupts the stem-loop structure is

(a) a first oligonucleotide selected from

(a-1) an oligonucleotide capable of binding to the nucleotide sequence complimentary to the nucleotide sequence of SEQ ID NO: 11, and
(a-2) an oligonucleotide capable of binding to the nucleotide sequence complimentary to the nucleotide sequence of SEQ ID NO: 12,

(b) a second oligonucleotide selected from

(b-1) an oligonucleotide capable of binding to the nucleotide sequence complimentary to the nucleotide sequence of SEQ ID NO: 13, and

(b-2) an oligonucleotide capable of binding to the nucleotide sequence complimentary to the nucleotide sequence of SEQ ID NO: 38,

(c) a third oligonucleotide selected from

(c-1) an oligonucleotide capable of binding to the nucleotide sequence complimentary to the nucleotide sequence of SEQ ID NO: 39, and
(c-2) an oligonucleotide capable of binding to the nucleotide sequence complimentary to the nucleotide sequence of SEQ ID NO: 16,
(d) a fourth oligonucleotide capable of binding to the nucleotide sequence complimentary to the nucleotide sequence of SEQ ID NO: 17,
(e) a combination of at least two oligonucleotides selected from the first oligonucleotide, the second oligonucleotide, and the third oligonucleotide,
or
(f) a combination of the first oligonucleotide and the fourth oligonucleotide.

[22] The composition according to any one of items 1 to 13 and 19 to 21, wherein the at least one substance that disrupts the stem-loop structure is

(a) a first oligonucleotide selected from
(b) a second oligonucleotide selected from

(a-1) an oligonucleotide comprising the nucleotide sequence of SEQ ID NO: 11, and
(a-2) an oligonucleotide comprising the nucleotide sequence of SEQ ID NO: 12,
(b-1) an oligonucleotide comprising the nucleotide sequence of SEQ ID NO: 13, and
(b-2) an oligonucleotide comprising the nucleotide sequence of SEQ ID NO: 38,

(c) a third oligonucleotide selected from

(c-1) an oligonucleotide comprising the nucleotide sequence of SEQ ID NO: 39, and
(c-2) an oligonucleotide comprising the nucleotide sequence of SEQ ID NO: 16,

(d) a fourth oligonucleotide comprising the nucleotide sequence of SEQ ID NO: 17,
(e) a combination of at least two oligonucleotides selected from the first oligonucleotide, the second oligonucleotide, and the third oligonucleotide,
or
(f) a combination of the first oligonucleotide and the fourth oligonucleotide.

[23] The composition according to any one of items 1 to 13 and 19 to 22, wherein the at least one substance that disrupts the stem-loop structure is

(a) a first oligonucleotide selected from

(a-1) an oligonucleotide consisting of the nucleotide sequence of SEQ ID NO: 11, and
(a-2) an oligonucleotide consisting of the nucleotide sequence of SEQ ID NO: 12,

(b) a second oligonucleotide selected from

(b-1) an oligonucleotide consisting of the nucleotide sequence of SEQ ID NO: 13, and
(b-2) an oligonucleotide consisting of the nucleotide sequence of SEQ ID NO: 38,

(c) a third oligonucleotide selected from

(c-1) an oligonucleotide consisting of the nucleotide sequence of SEQ ID NO: 39, and
(c-2) an oligonucleotide consisting of the nucleotide sequence of SEQ ID NO: 16,

(d) a fourth oligonucleotide consisting of the nucleotide sequence of SEQ ID NO: 17,
(e) a combination of at least two oligonucleotides selected from the first oligonucleotide, the second oligonucleotide-

tide, and the third oligonucleotide,
or
(f) a combination of the first oligonucleotide and the fourth oligonucleotide.

[24] The composition according to any one of items 13 to 23, wherein any one of the first to third oligonucleotides consists of 15 to 25, preferably 18 to 22, more preferably 19 to 21 nucleotides.
[25] The composition according to any one of items 13 to 24, wherein the fourth oligonucleotide consists of 25 to 30, preferably 25 to 27 nucleotides.
[26] The composition according to any one of items 13 to 25, wherein the at least one substance that disrupts the stem-loop structure is the combination of the second oligonucleotide and the third oligonucleotide.
[27] The composition according to any one of items 13 to 25, wherein the at least one substance that disrupts the stem-loop structure is the fourth oligonucleotide.
[28] The composition according to any one of items 2 to 27, wherein the cancer is malignant melanoma, non-small cell lung cancer, small cell lung cancer, renal cell carcinoma, classical Hodgkin lymphoma, head and neck cancer, gastric cancer, malignant pleural mesothelioma, colorectal cancer, esophageal cancer, cancer of unknown primary, urothelial carcinoma, hepatocellular carcinoma, solid tumor, breast cancer, endometrial cancer, malignant pleural mesothelioma, Merkel cell carcinoma, brain tumor, bile duct cancer, renal cancer, or colon cancer.
[29] The composition according to any one of items 2 to 28, wherein the cancer is malignant melanoma, non-small cell lung cancer, small cell lung cancer, renal cell carcinoma, classical Hodgkin lymphoma, head and neck cancer, gastric cancer, malignant pleural mesothelioma, colorectal cancer, esophageal cancer, cancer of unknown primary, urothelial carcinoma, hepatocellular carcinoma, solid tumor, breast cancer, endometrial cancer, malignant pleural mesothelioma, or Merkel cell carcinoma.
[30] The composition according to any one of items 2 to 28, wherein the cancer is brain tumor or bile duct cancer.
[31] The composition according to any one of items 2 to 28, wherein the cancer is malignant melanoma, renal cancer, or colon cancer.
[32] The composition according to any one of items 2 to 28 and 31, wherein the cancer is malignant melanoma.
[33] A method of screening for an agent for treating cancer, comprising

(a) introducing a candidate substance to a cell expressing Regnase-1 and/or Regnase-3 and a reporter gene fused with the 3' untranslated region of an Nfkbiz mRNA;
(b) measuring the expression level of the reporter gene in the cell, and
(c) identifying the candidate substance as the agent for treating cancer when the expression level measured in the presence of the candidate substance is higher than the expression level measured in the absence of the candidate substance.

[34] The method according to item 33, wherein the 3' untranslated region of the Nfkbiz mRNA comprises a nucleotide sequence having at least about 90% identity with the nucleotide sequence of SEQ ID NO: 5.
[35] The method according to item 33 or 34, wherein the 3' untranslated region of the Nfkbiz mRNA comprises the nucleotide sequence of SEQ ID NO: 5.
[36] The method according to any one of items 33 to 35, wherein the 3' untranslated region of the Nfkbiz mRNA comprises a nucleotide sequence having at least about 90% identity with the nucleotide sequence of SEQ ID NO: 7.
[37] The method according to any one of items 33 to 36, wherein the 3' untranslated region of the Nfkbiz mRNA comprises the nucleotide sequence of SEQ ID NO: 7.
[38] The method according to any one of items 33 to 37, wherein the 3' untranslated region of the Nfkbiz mRNA consists of a nucleotide sequence having at least about 90% identity with the nucleotide sequence of SEQ ID NO: 9.
[39] The method according to any one of items 33 to 38, wherein the 3' untranslated region of the Nfkbiz mRNA consists of the nucleotide sequence of SEQ ID NO: 9.
[40] The method according to any one of items 33 to 39, wherein the candidate substance is a substance designed based on a stem-loop structure in the 3' untranslated region of the Nfkbiz mRNA.
[41] A substance that disrupts a stem-loop structure in the 3' untranslated region of an Nfkbiz mRNA.
[42] A method of stimulating an immune cell, comprising introducing in vitro at least one substance that disrupts a stem-loop structure in the 3' untranslated region of an Nfkbiz mRNA to the immune cell.
[43] An immune cell, comprising at least one substance that disrupts a stem-loop structure in the 3' untranslated region of an Nfkbiz mRNA.
[44] A composition for treating cancer, comprising an immune cell comprising at least one substance that disrupts a stem-loop structure in the 3' untranslated region of an Nfkbiz mRNA.

The entire contents of the documents cited herein are incorporated herein by reference.

The following examples are non-limiting and provided only for describing the invention. The embodiments described above are non-limiting and may be modified without deviating from the scope of the invention as defined by the appended claims.

EXAMPLE 1

[Materials and Methods]

Design of oligonucleotides

Morpholino oligonucleotides of the following sequences were prepared by GeneTools, LLC.

Mouse Nfkbiz SL2-targeting morpholino oligo (SL2-mNfkbiz-MO); 5'-taactgacagtgagtgtcgc-3' (SEQ ID NO: 18)
Mouse Nfkbiz SL3-targeting morpholino oligo (SL3-mNfkbiz-MO); 5'-aatacagggcaaatggtcta-3' (SEQ ID NO: 31)
Mouse Nfkbiz SL4-targeting morpholino oligo (SL4-ASO); 5'-aactaaatgggtttgtttc-3' (SEQ ID NO: 40)
Mouse Nfkbiz SL5-targeting morpholino oligo (SL5-ASO); 5'-atagtgaactaaatgggt-3' (SEQ ID NO: 41)
Mouse Nfkbiz SL45-targeting morpholino oligo (SL45-mNfkbiz-MO, Nfkbiz-ASO, SL4/5-ASO) ;5'-atagtgaactaaatg ggtttgtttc-3' (SEQ ID NO: 22)
Human NFKBIZSL45-targeting morpholino oligo (SL45-hNFKBIZ-MO); 5'-atagtgaactaaatatgtttgtttc-3' (SEQ ID NO: 17)

Luciferase reporter assay

HeLa cells were seeded in 24-well plates at $1x10^4$ cells/well and after 24 hours a control oligonucleotide (Standard Control, GeneTools, LLC) or an Nfkbiz-targeting oligonucleotide was introduced by transfection using Endo-Porter (GeneTools, LLC) (final concentration: 3 μM). Immediately, pGL3-luciferase (with no inserted sequence) or the vector having mouse Nfkbiz 3' UTR sequence inserted downstream of the luciferase gene was transfected with Lipofectamine LTX Reagent (Thermo Fisher scientific). The cells were collected after 24 hours. Luciferase activities of the cell lysates were measured by Dual-Luciferase Reporter Assay System (Promega). The luciferase activities were standardized with Renilla luciferase gene.

Mice

Wild-type C57BL/6JJcl mice were purchased from CLEA Japan, Inc. Regnase-1 flox mice (Uehata, T., et al. (2013). Cell 153, 1036-1049), Nfkbiz flox mice (Okuma A. et al., Immunity 2013 Mar 21;38(3):450-60), CreERT2 mice (Ventura A., et al., Nature 2007 Feb 8;445(7128): 661-5), and OT-I mice (Hogquist K.A., et al., Cell 1994 Jan 14;76(1):17-27) have been reported in previous studies. Regnase-3 knockout mice were generated by genome editing using the CRISPR-Cas9 method. Mice were bred under SPF conditions and 8-12 weeks old mice were used for analysis.

Isolation of naive CD8-positive T cells

Spleens and lymph nodes from C57BL/6JJcl or OT-I mice were harvested and grinded in staining buffer (PBS (pH 7.2) containing 0.5% BSA and 2 mM EDTA) to give cell suspensions. Erythrocytes were lysed with ACK lysis buffer (Gibco). Biotin-anti-CD4 antibody was added to Pan T Cell Isolation Kit II (Miltenyi Biotec) and CD8-positive T cells were isolated by negative selection. The cells were then stained with PE-anti-CD44 antibody, followed by negative selection using Anti-PE MicroBeads (Miltenyi Biotec), and the resulting cells were isolated as naive CD8-positive T cells.

Transfection of antisense nucleic acids

Mouse naive CD8-positive T cells were seeded in 24-well plates coated with Hamster IgG (MP Biochemicals) at $2x10^5$ cells/well and stimulated with anti-CD3ε antibody (final concentration: 0.25 μg/mL) and anti-CD28 antibody (final concentration: 1.0 μg/mL) for 24 hours. Activated T cells were collected, washed with PBS, and transfected with a control nucleic acid or an Nfkbiz-targeting nucleic acid by electroporation using P3 Primary Cell 4D-NucleofectorTM X Kit (Lonza). The cells were then transferred to T-flasks and incubated in RPMI (2-mercaptoethanol, penicillin-streptomycin, 10% FCS) containing IL-2 (10 ng/mL), IL-7 (2.5 ng/mL), and IL-15 (25 ng/mL) for 3 days.

Isolation of bone marrow derived macrophages and transfection of oligonucleotides

Femurs, tibias, and sacrums were harvested from wild-type C57BL/6JJcl mice, grinded in RPMI (2-mercaptoethanol, penicillin-streptomycin, 10% FCS) solution, and cells were collected by filtration with a cell strainer (100 μM). After centrifugation, erythrocytes were lysed by applying ACK lysis buffer (Gibco) to the cell pellet. After washing once with RPMI, the bone marrow cells were cultured in the presence of M-CSF (20 ng/mL) for 6 days to isolate bone marrow derived macrophages. On day 6 cells were collected from the plates with PBS containing 10 mM EDTA, further washed with PBS, and transfected with a control oligonucleotide or an Nfkbiz-targeting oligonucleotide by electroporation using P2 Primary Cell 4D-NucleofectorTM X Kit (Lonza). The cells were then transferred to 24-well plates and cultured in RPMI containing M-CSF (20 ng/mL) for 24 hours.

Measurement of cytokines and cytotoxic substances

The T cells transfected with the nucleic acids were collected, washed with PBS, and seeded in 96-well U-bottomed plates. The cells were stimulated with IL-12 (10 ng/mL) and IL-18 (1.0 ng/mL) in the presence of Golgi stop (BD Biosciences) for 4 hours. After the stimulation, the cells were collected and anti-CD8a and anti-TCRβ were used for staining the cell surface antigens. The cells were then fixed with fixation/permeabilization buffer, and IFNγ, granzyme-b, and TNF were stained intracellularly. Cytokines were measured by FACS (BD LSRFortessa).

Cancer killing assay

Ovalbumin-expressing B16 cancer cell line (B16-OVA) was cultured in DMEM containing penicillin-streptomycin and 10% FCS. The culture medium was replaced with RPMI containing 2-mercaptoethanol, penicillin-streptomycin, and 10% FCS, and the cells were seeded in 96-well flat-bottomed plates at $2x10^4$ cells/well. The T cells transfected with the nucleic acids were then collected, washed with PBS, and co-cultured with the B16-OVA cells in the 96-well plates at a ratio of 1:1 to 1:16 relative to the number of the B16-OVA cells, and the culture supernatants were collected after 4 hours. The OD490 value of the culture medium was measured by CytoTox96 Non-Radioactive Cytotoxicity Assay (Promega). The OD490 value measured in the T cell-free control group was considered as background and subtracted from each OD490 value. Anti-tumor activity was determined as % cytotoxicity, where the maximum OD490 value was assumed to be observed when all cancer cells were killed.

[Results]

Test 1: Reg1/3-Nfkbiz regulatory axis plays an important role in regulating T cell activation

Nfkbiz mRNA is an important target gene of Regnase-1 and Regnase-3. To reveal the role of Nfkbiz as a Regnase target gene in vivo, the following analysis was performed by using tamoxifen-induced gene deletion system in CreERT2 mice. Gene deletion was generated by intraperitoneal administration of 2 mg tamoxifen/100 μL of corn oil for 5 consecutive days and analysis was carried out on day 14. Fig. 1 shows the genotypes used for analysis and the experimental schedule. Mice with double deficiency of Regnase-1 and Regnase-3 (DKO) showed marked splenomegaly. However, mice lacking Nfkbiz in addition to Regnase-1 and Regnase-3 (TKO) as well as the wild type mice showed no splenomegaly (Fig. 2). Next, splenocytes collected from mice were analyzed by flow cytometry (FACS). Fig. 3 shows $CD62L^+CD44^-$ naive T cells and $CD62L^-CD44^+$ effector T cells in CD4-positive T cells, as well as CD69 expression, which is an activation marker. In splenocytes from DKO, effector T cells were increased and CD69 expression was higher compared to those from wild-type mice. However, in TKO mice these phenotypes were not different from those of the wild-type mice, and no splenomegaly was observed. Similar results were observed for CD8-positive T cells (Fig. 4). $CD62L^-CD44^+$ effector T cells were significantly increased in splenocytes from DKO mice, but no difference from controls was observed in splenocytes from TKO mice. The results suggest that Regnase-1 and Regnase-3 regulate T cell activation by suppressing Nfkbiz expression.

Test 2: Strategy for immunostimulation by targeting unstable elements in Nfkbiz mRNA

Nucleotide sequences of the 3' untranslated region of Nfkbiz gene from various species were aligned. Fig. 5 shows the alignment of nucleotides at positions 29-148 and the regions capable of forming secondary structures (SL2-5). Fig. 6 is a schematic drawing showing the stem-loop structures (SL4 and 5) in the 3' untranslated region of mouse Nfkbiz mRNA and disruption of the secondary structures by antisense nucleotides targeting the structures. As shown in the right panel of Fig. 6, the antisense nucleic acid that complementarily binds to the region from a part of SL4 (3' stem portion of SL4) to a part of SL5 (5' stem portion of SL5) in the 3' untranslated region of mouse Nfkbiz mRNA is referred to as SL45-mNfkbiz-MO.

Luciferase reporter assay was performed using pGL3-luciferase having mouse Nfkbiz 3' UTR sequence inserted downstream of the luciferase gene. Fig. 7 shows the method and results. The luciferase activity was significantly higher in HeLa cells transfected with Nfkbiz-targeting morpholino oligos targeting SL2 or SL4 and 5 (SL2-mNfkbiz-MO or SL45-mNfkbiz-MO) than in HeLa cells transfected with the control morpholino oligo (control-MO). No difference in luciferase activity was observed between HeLa cells transfected with Nfkbiz-MO targeting SL3 (SL3-mNfkbiz-MO) and HeLa cells transfected with control-MO.

Similar luciferase reporter assay was performed using pGL3-luciferase having human Nfkbiz 3' UTR sequence inserted downstream of the luciferase gene. Fig. 8 shows the method and results. Luciferase activity was significantly higher in HeLa cells transfected with Nfkbiz-targeting morpholino oligo targeting SL4 and 5 (SL45-hNfkbiz-MO) than in HeLa cells transfected with the control morpholino oligo (control-MO).

The results suggest that disruption of a specific stem-loop structure in the 3' untranslated region of Nfkbiz gene increases Nfkbiz expression. Fig. 9 is a schematic drawing showing Nfkbiz gene expression is modified by an antisense nucleic acid.

Test 3: Nucleic acid drugs targeting Nfkbiz suppress cancer

Fig. 10 is a schematic drawing showing anti-tumor effects of cytotoxic T cells. As outlined in Fig. 11, OT-I CD8-positive T cells were transfected with SL45-mNfkbiz-MO or control-MO, cultured for 72 hours, and subjected to cytokine measurement and cancer killing assay.

Expression levels of IFN$\gamma$, granzyme-b, and TNF were evaluated by FACS. Fig. 12 shows the percentages (%) (upper panel) and mean fluorescence intensities (MFI) (lower panel) of CD8T cells producing IFN$\gamma$, granzyme-b, and TNF. In the cells transfected with SL45-mNfkbiz-MO, the expression levels of IFN$\gamma$ and granzyme-b were significantly higher than in controls.

Fig. 13 shows the results of the cancer killing assay. The antitumor activity of OT-I CD8T cells against B16-OVA cell line (melanoma cell line) was compared between the cells transfected with SL45-mNfkbiz-MO and the cells transfected with control-MO. The cytotoxicity was higher in the cells transfected with SL45-mNfkbiz-MO than in the cells transfected with control-MO and increased in a dose-dependent manner.

Test 4: Nfkbiz-targeting nucleic acids continuously enhance Nfkbiz protein expression in CD8-positive T cells

$CD62L^+CD44^-$ naive CD8-positive T cells isolated from spleens and lymph nodes of wild-type C57BL/6JJcl mice were stimulated with anti-CD3$\varepsilon$ antibody (final concentration: 0.25 $\mu$g/mL) and anti-CD28 antibody (final concentration: 1.0 $\mu$g/mL) for 24 hours. The cells were transfected with Nfkbiz-ASO or a control oligonucleotide (Control-oligo) at 10 $\mu$M or 50 $\mu$M by electroporation. The changes in Nfkbiz expression over time after ASO transfection were evaluated by Western blotting. Fig. 14 shows the results. Nfkbiz-ASO continuously enhanced Nfkbiz protein expression in CD8-positive T cells.

Test 5: Nfkbiz-targeting nucleic acids increase Nfkbiz expression in macrophages and enhance inflammatory responses

Bone marrow-derived macrophages from wild-type C57BL/6JJcl mice were transfected with 50 $\mu$M of Nfkbiz-ASO or Control-oligo and stimulated with LPS (100 ng/mL). Nfkbiz expression after the stimulation was quantified over time by qPCR. Nfkbiz expression at 24 hours was also evaluated by Western blotting. Figs. 15 and 16 show the results. Nfkbiz-ASO continuously enhanced Nfkbiz expression in bone marrow-derived macrophages.

Similarly, mouse bone marrow-derived macrophages were transfected with Nfkbiz-ASO or Control-oligo and stimulated with LPS (100 ng/mL). Actinomycin D (5 $\mu$g/mL) was added 2 hours after the LPS stimulation and subsequent Nfkbiz expression was evaluated by qPCR. Fig. 17 shows the results. Nfkbiz-ASO suppressed the attenuation of Nfkbiz expression in the presence of the transcriptional inhibitor. The results suggest that the increase in Nfkbiz expression by Nfkbiz-ASO depends on suppression of Nfkbiz degradation.

Similarly, mouse bone marrow-derived macrophages were transfected with Nfkbiz-ASO or Control-oligo and stimulated with LPS (100 ng/mL) for 24 hours. IL-6 levels in the culture supernatants were evaluated by ELISA. Fig. 18 shows the results. The results suggest that Nfkbiz-ASO increases IL-6 production and enhances inflammatory responses.

Mouse bone marrow-derived macrophages were transfected with 50 $\mu$M of SL4-mNfkbiz-MO (SL4-ASO), SL5-mNfkbiz-MO (SL5-ASO), SL45-mNfkbiz-MO (SL4/5-ASO), or Control-oligo by electroporation. Fig. 19 shows schematic drawings of the MOs. Twenty-four hours after the transfection, the expression levels of Nfkbiz were evaluated by qPCR and Western blotting. Figs. 20 and 21 show the results. The morpholino oligos targeting SL4 or SL5 used alone tended to inhibit Nfkbiz degradation. The morpholino oligo that disrupts both of SL4 and SL5 suppressed Nfkbiz degradation very strongly.

Test 6: Cytotoxic T cells transfected with Nfkbiz-ASO produce tumor regression in vivo

C57BL/6J mice were inoculated subcutaneously with $5 \times 10^5$ cells of B16-OVA cell line in the lateral abdomen. Ovalbumin (OVA)-specific naive CD8-positive T cells were isolated from spleens and lymph nodes of OT-I+Rosa-E2a-Td-tomato+RAG2$^{-/-}$ mice and stimulated with anti-CD3ε antibody/anti-CD28 antibody. Twenty-four hours after the stimulation, the cells were transfected with Nfkbiz-ASO or Control-oligo by electroporation, and cultured in RPMI medium containing IL-2/IL-7/IL-15 for 4 days. On day 7 of the tumor inoculation, $5 \times 10^6$ cells/200 μL PBS of the T cells or 200 μL of PBS (control) were transferred from the orbital vein to mice having a similar tumor size (N=3). The tumor sizes were measured over time. Fig. 22 shows the workflow of the test. Fig. 23 shows the results. Tumor growth was inhibited in the mice treated with the cytotoxic T cells transfected with Nfkbiz-ASO.

Test 7: Enhanced Nfkbiz expression by Nfkbiz-ASO inhibits T cell exhaustion

CD8-positive T cells from C57BL/6JJcl mice were transfected with Nfkbiz-ASO by electroporation and cultured in the presence of IL-2/IL-7/IL-15 for 4 days. The cells were collected and expressions of PD-1, LAG-3, TOX, and TCF-1 were evaluated by flow cytometry. PD-1, LAG-3, and TOX are markers of T cell exhaustion, and TCF-1 is a marker of stem cell-like memory T cells. Fig. 24 shows the results. The numbers in the figure indicate the percentage of cells present in the framed area (%). The PD-1-positive/LAG-3-positive cell populations (cells in the frames of the upper panels) and TOX-positive/TCF1-positive cell populations (cells in the frames of the lower panels) were lower in the T cells transfected with Nfkbiz-ASO (upper right and lower right panels) than in the T cells transfected with Control-oligo (upper left and lower left panels). These results indicate that T cell exhaustion can be inhibited by enhanced Nfkbiz expression by Nfkbiz-ASO.

Test 8: Increased expression of NFKBIZ in PBMCs transfected with human NFKBIZ-ASO

Human peripheral blood mononuclear cells (PBMCs) were transfected with Nfkbiz-ASO by electroporation, and the expression of NFKBIZ protein in the PBMCs was analyzed by Western blotting. Fig. 25 shows the results. The expression of NFKBIZ protein was higher in the PBMCs transfected with Nfkbiz-ASO than in the PBMCs transfected with Control-oligo.

These results suggest that disruption of specific stem-loop structures in the 3' untranslated region of Nfkbiz gene enhances the antitumor activity of cytotoxic T cells.

INDUSTRIAL APPLICABILITY

The disclosure provides the method of immunostimulation and the method of treating cancer, which are useful in the field of medicine.

## Claims

1. A composition for treating cancer, comprising at least one substance that disrupts a stem-loop structure in the 3' untranslated region of an Nfkbiz mRNA,

    wherein the stem-loop structure is at least one stem-loop structure selected from the following stem-loop structures;
    a first stem-loop structure formed in a region corresponding to positions 34 to 50 of SEQ ID NO: 9,
    a second stem-loop structure formed in a region corresponding to positions 100 to 116 of SEQ ID NO: 9, and
    a third stem-loop structure formed in a region corresponding to positions 129 to 143 of SEQ ID NO: 9.

2. The composition according to claim 1, wherein the composition comprises at least one substance that disrupts at least one stem-loop structure selected from the second stem-loop structure and the third stem-loop structure.

3. The composition according to claim 1, wherein the composition comprises at least one substance that disrupts the second stem-loop structure and the third stem-loop structure.

4. The composition according to claim 1, wherein the 3' untranslated region of the Nfkbiz mRNA comprises a nucleotide sequence having at least about 90% identity with the nucleotide sequence of SEQ ID NO: 5.

5. The composition according to claim 1, wherein the 3' untranslated region of the Nfkbiz mRNA comprises a nucleotide

sequence having at least about 90% identity with the nucleotide sequence of SEQ ID NO: 7.

**6.** The composition according to claim 1, wherein the at least one substance that disrupts the stem-loop structure is

(a) a first oligonucleotide selected from

(a-1) an oligonucleotide consisting of 10 to 30 nucleotides and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of 10 to 30 nucleotides and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of 10 to 30 nucleotides and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of 10 to 30 nucleotides and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of 10 to 30 nucleotides and being capable of binding to a sequence that consists of contiguous 10 to 30 nucleotides within positions 105 to 139 of SEQ ID NO: 9 and comprises the nucleotides at positions 132 to 134 of SEQ ID NO: 9, and
(c-2) an oligonucleotide consisting of 10 to 30 nucleotides and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of 25 to 35 nucleotides and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

**7.** The composition according to claim 1, wherein the at least one substance that disrupts the stem-loop structure is

(a) a first oligonucleotide selected from

(a-1) an oligonucleotide consisting of a nucleotide sequence having at least 90% identity with SEQ ID NO: 11 and being capable of binding to a sequence that consists of contiguous 10 to 30 nucleotides within positions 8 to 48 of SEQ ID NO: 9 and comprises the nucleotides at positions 35 to 37 of SEQ ID NO: 9, and
(a-2) an oligonucleotide consisting of a nucleotide sequence having at least 90% identity with SEQ ID NO: 12 and being capable of binding to a sequence that consists of contiguous 10 to 30 nucleotides within positions 36 to 76 of SEQ ID NO: 9 and comprises the nucleotides at positions 47 to 49 of SEQ ID NO: 9,

(b) a second oligonucleotide selected from

(b-1) an oligonucleotide consisting of a nucleotide sequence having at least 90% identity with SEQ ID NO: 13 and being capable of binding to a sequence that consists of contiguous 10 to 30 nucleotides within positions 77 to 111 of SEQ ID NO: 9 and comprises the nucleotides at positions 104 to 106 of SEQ ID NO: 9, and
(b-2) an oligonucleotide consisting of a nucleotide sequence having at least 90% identity with SEQ ID NO: 38 and being capable of binding to a sequence that consists of contiguous 10 to 30 nucleotides within positions 105 to 139 of SEQ ID NO: 9 and comprises the nucleotides at positions 110 to 112 of SEQ ID NO: 9,

(c) a third oligonucleotide selected from

(c-1) an oligonucleotide consisting of a nucleotide sequence having at least 90% identity with SEQ ID NO: 39 and being capable of binding to a sequence that consists of contiguous 10 to 30 nucleotides within positions 105 to 139 of SEQ ID NO: 9 and comprises the nucleotides at positions 132 to 134 of SEQ ID NO: 9, and
(c-2) an oligonucleotide consisting of a nucleotide sequence having at least 90% identity with SEQ ID NO: 16 and being capable of binding to a sequence that consists of contiguous 10 to 30 nucleotides within positions 133 to 167 of SEQ ID NO: 9 and comprises the nucleotides at positions 138 to 140 of SEQ ID NO: 9,

(d) a fourth oligonucleotide consisting of a nucleotide sequence having at least 90% identity with SEQ ID NO: 17 and being capable of binding to a sequence that consists of contiguous 25 to 35 nucleotides within positions 105 to 139 of SEQ ID NO: 9 and comprises the nucleotides at positions 110 to 134 of SEQ ID NO: 9,
(e) a combination of at least two oligonucleotides selected from the first oligonucleotide, the second oligonucleotide, and the third oligonucleotide,
or
(f) a combination of the first oligonucleotide and the fourth oligonucleotide.

**8.** The composition according to claim 6, wherein the at least one substance that disrupts the stem-loop structure is the combination of the second oligonucleotide and the third oligonucleotide.

**9.** The composition according to claim 6, wherein the at least one substance that disrupts the stem-loop structure is the fourth oligonucleotide.

**10.** The composition according to any one of claims 1 to 9, wherein the cancer is malignant melanoma, non-small cell lung cancer, small cell lung cancer, renal cell carcinoma, classical Hodgkin lymphoma, head and neck cancer, gastric cancer, malignant pleural mesothelioma, colorectal cancer, esophageal cancer, cancer of unknown primary, urothelial carcinoma, hepatocellular carcinoma, solid tumor, breast cancer, endometrial cancer, malignant pleural mesothelioma, Merkel cell carcinoma, brain tumor, bile duct cancer, renal cancer, or colon cancer.

**11.** A composition for immunostimulation, comprising at least one substance that disrupts a stem-loop structure in the 3' untranslated region of an Nfkbiz mRNA,

wherein the stem-loop structure is at least one stem-loop structure selected from the following stem-loop structures;
a first stem-loop structure formed in a region corresponding to positions 34 to 50 of SEQ ID NO: 9,
a second stem-loop structure formed in a region corresponding to positions 100 to 116 of SEQ ID NO: 9, and
a third stem-loop structure formed in a region corresponding to positions 129 to 143 of SEQ ID NO: 9.

[Fig. 1]

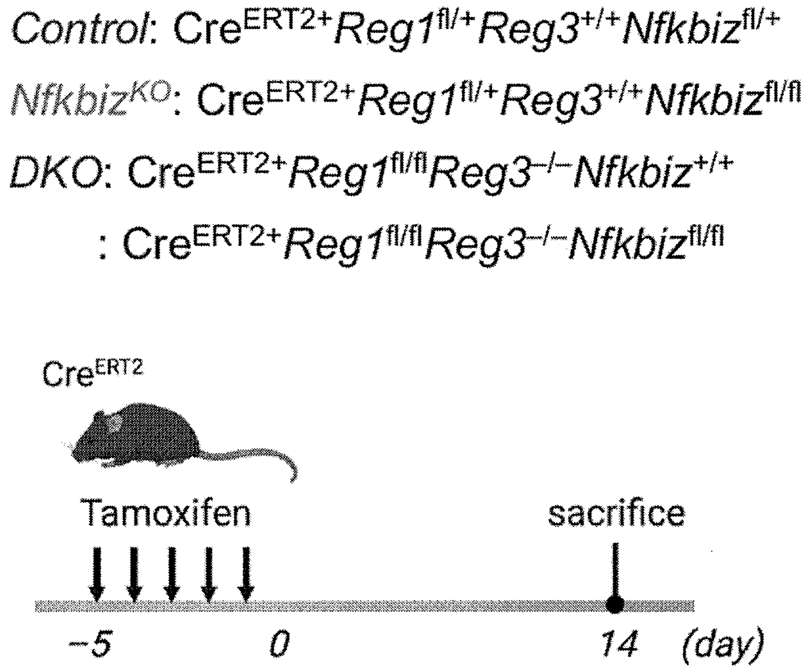
Control: Cre$^{ERT2+}$Reg1$^{fl/+}$Reg3$^{+/+}$Nfkbiz$^{fl/+}$
Nfkbiz$^{KO}$: Cre$^{ERT2+}$Reg1$^{fl/+}$Reg3$^{+/+}$Nfkbiz$^{fl/fl}$
DKO: Cre$^{ERT2+}$Reg1$^{fl/fl}$Reg3$^{-/-}$Nfkbiz$^{+/+}$
: Cre$^{ERT2+}$Reg1$^{fl/fl}$Reg3$^{-/-}$Nfkbiz$^{fl/fl}$
Cre$^{ERT2}$
Tamoxifen
sacrifice
−5
0
14
(day)

[Fig. 2]

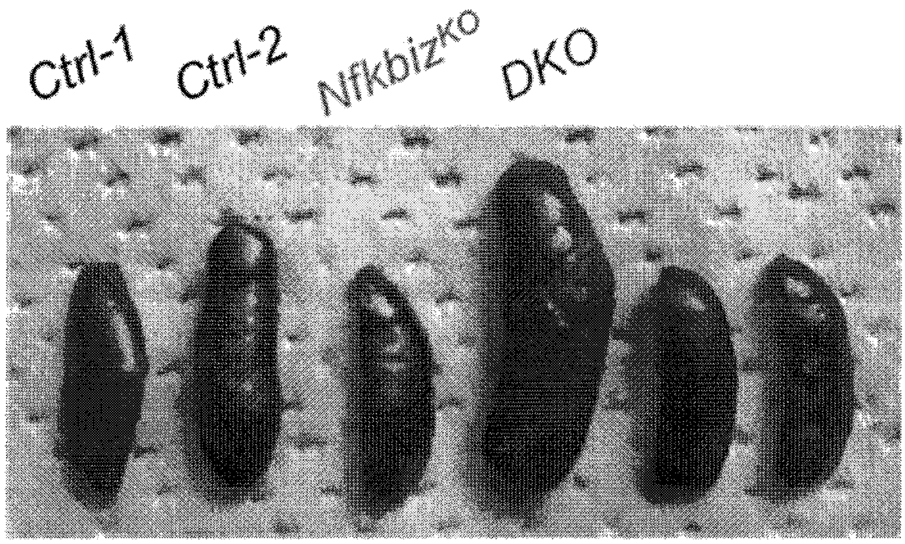
Ctrl-1
Ctrl-2
Nfkbiz$^{KO}$
DKO

[Fig. 3]

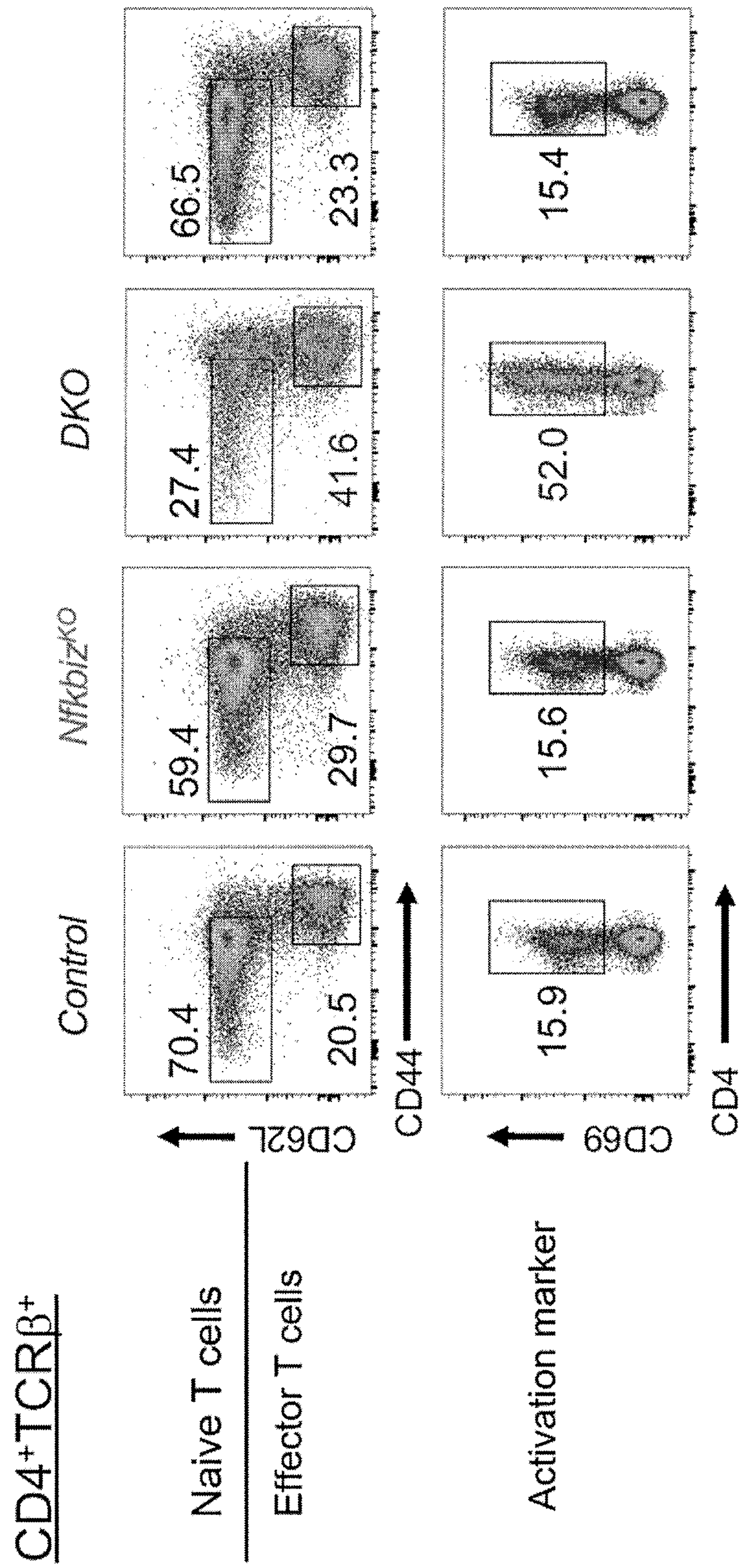
CD4+TCRβ+
Naive T cells
Effector T cells
Activation marker
Control
NfkbizKO
DKO
CD62L
CD44
CD69
CD4
70.4
20.5
59.4
29.7
27.4
41.6
66.5
23.3
15.9
15.6
52.0
15.4

[Fig. 4]

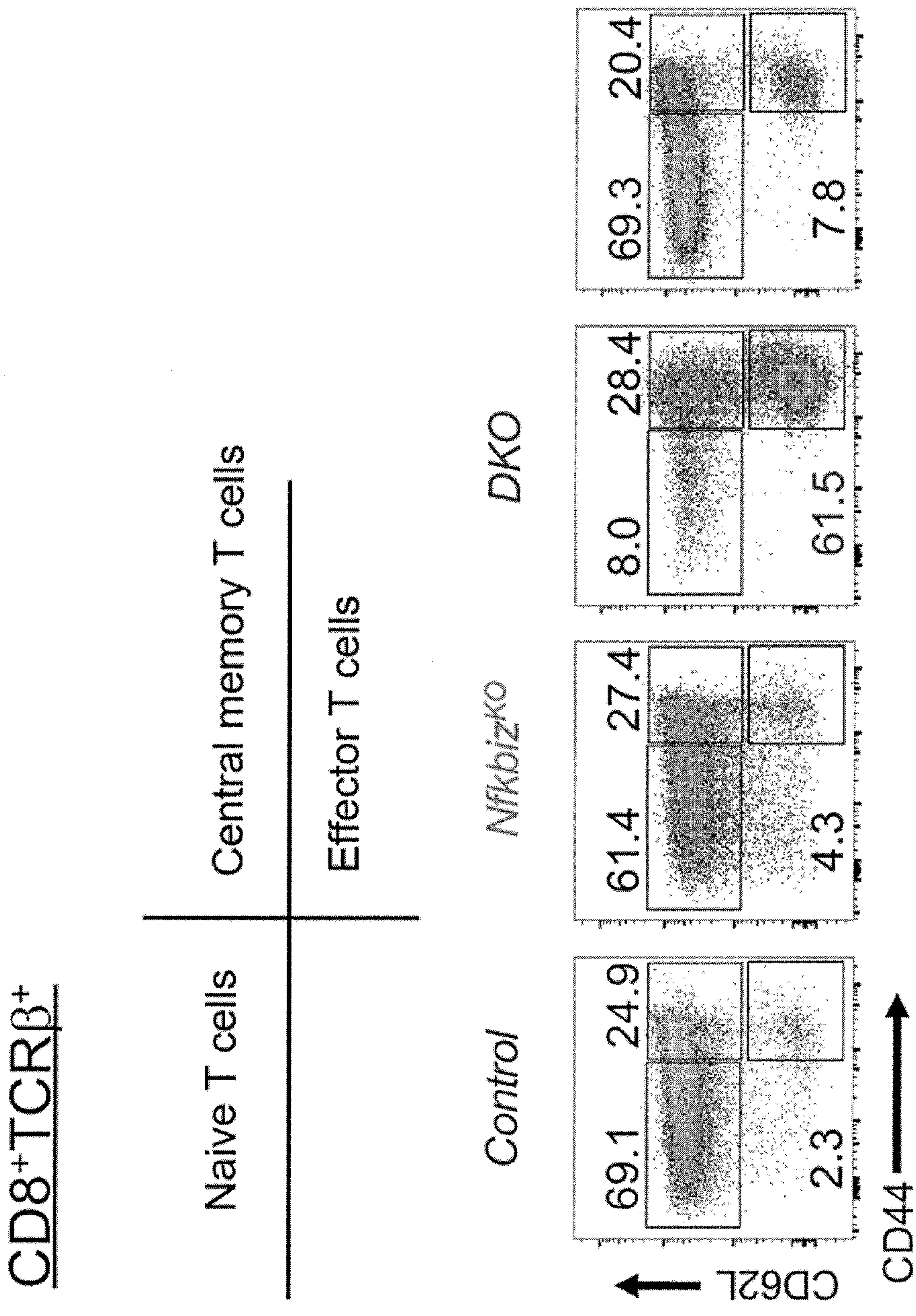
CD8+TCRβ+
Naive T cells
Central memory T cells
Effector T cells
Control
NfkbizKO
DKO
69.1
24.9
2.3
61.4
27.4
4.3
8.0
28.4
61.5
69.3
20.4
7.8
CD62L
CD44

[Fig. 5]

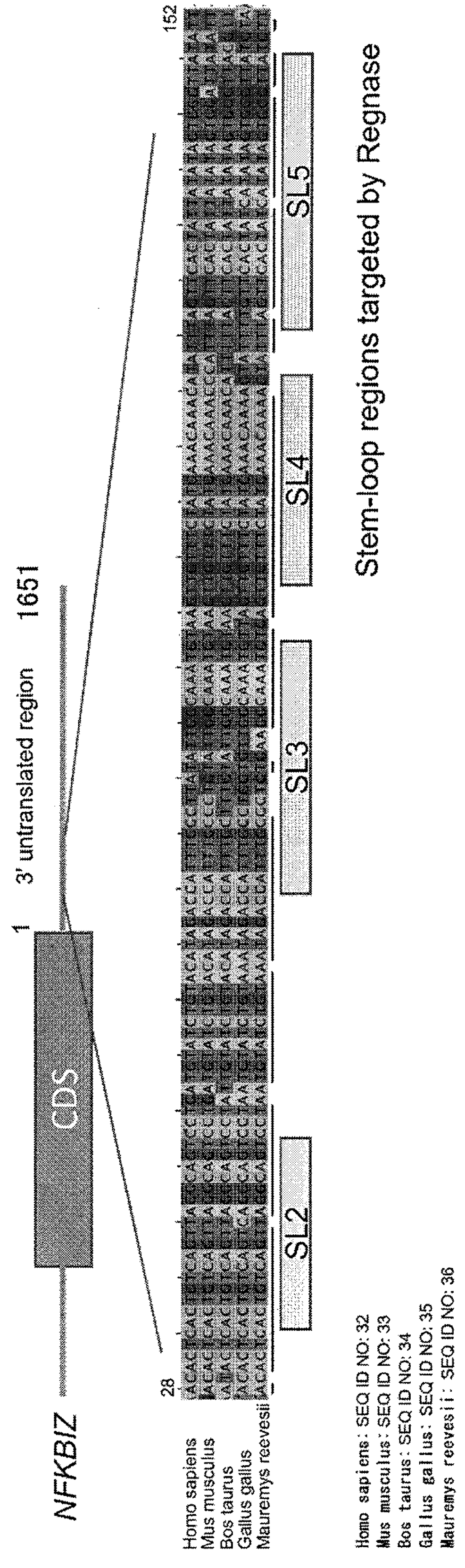
NFKBIZ
CDS
1
3' untranslated region
1651
28
152
Homo sapiens
Mus musculus
Bos taurus
Gallus gallus
Mauremys reevesii
SL2
SL3
SL4
SL5
Stem-loop regions targeted by Regnase
Homo sapiens: SEQ ID NO: 32
Mus musculus: SEQ ID NO: 33
Bos taurus: SEQ ID NO: 34
Gallus gallus: SEQ ID NO: 35
Mauremys reevesii: SEQ ID NO: 36

[Fig. 6]

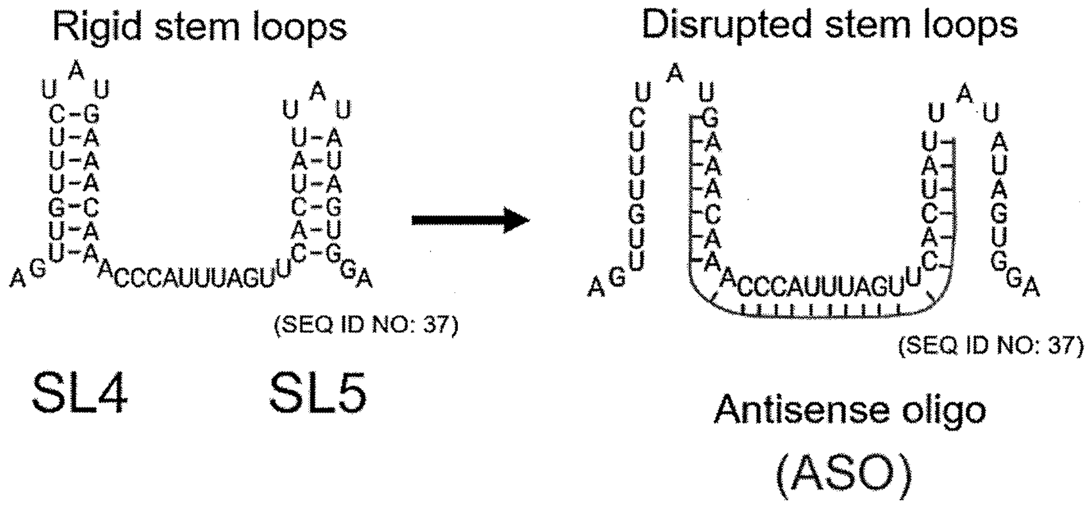
Rigid stem loops
Disrupted stem loops
ACCCAUUUAGUU
(SEQ ID NO: 37)
SL4
SL5
(SEQ ID NO: 37)
Antisense oligo
(ASO)

[Fig. 7]

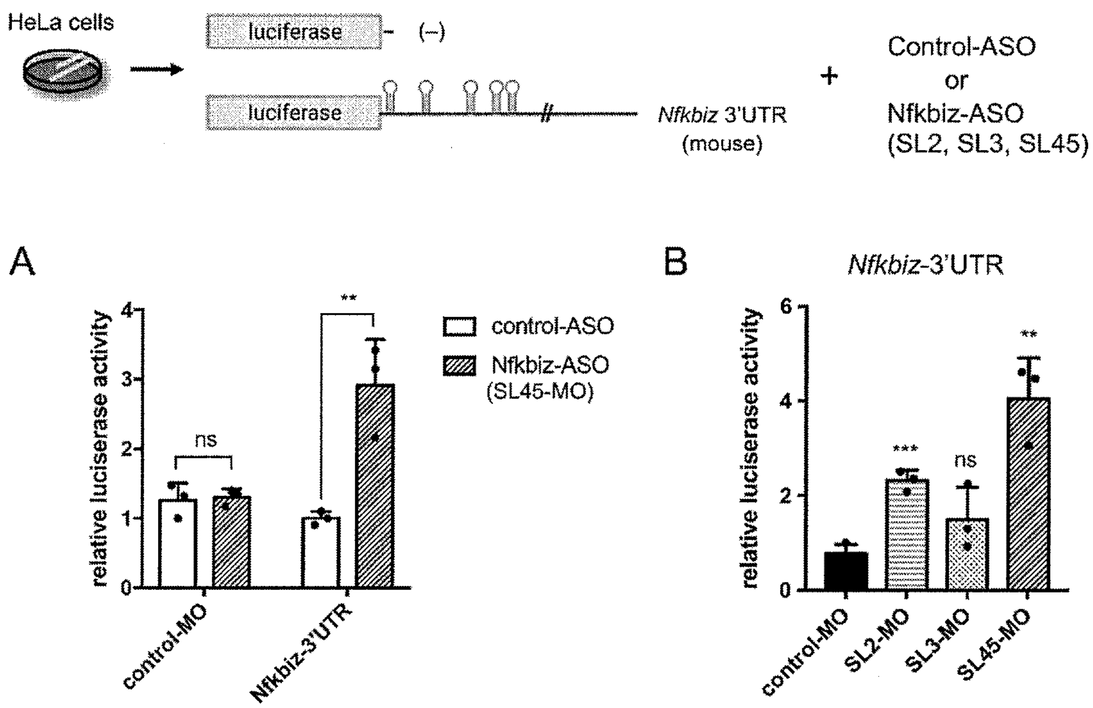
HeLa cells
luciferase
(–)
luciferase
Nfkbiz 3'UTR
(mouse)
+
Control-ASO
or
Nfkbiz-ASO
(SL2, SL3, SL45)
A
relative luciferase activity
ns
**
control-ASO
Nfkbiz-ASO
(SL45-MO)
control-MO
Nfkbiz-3'UTR
B
Nfkbiz-3'UTR
relative luciferase activity
***
ns
**
control-MO
SL2-MO
SL3-MO
SL45-MO

[Fig. 8]

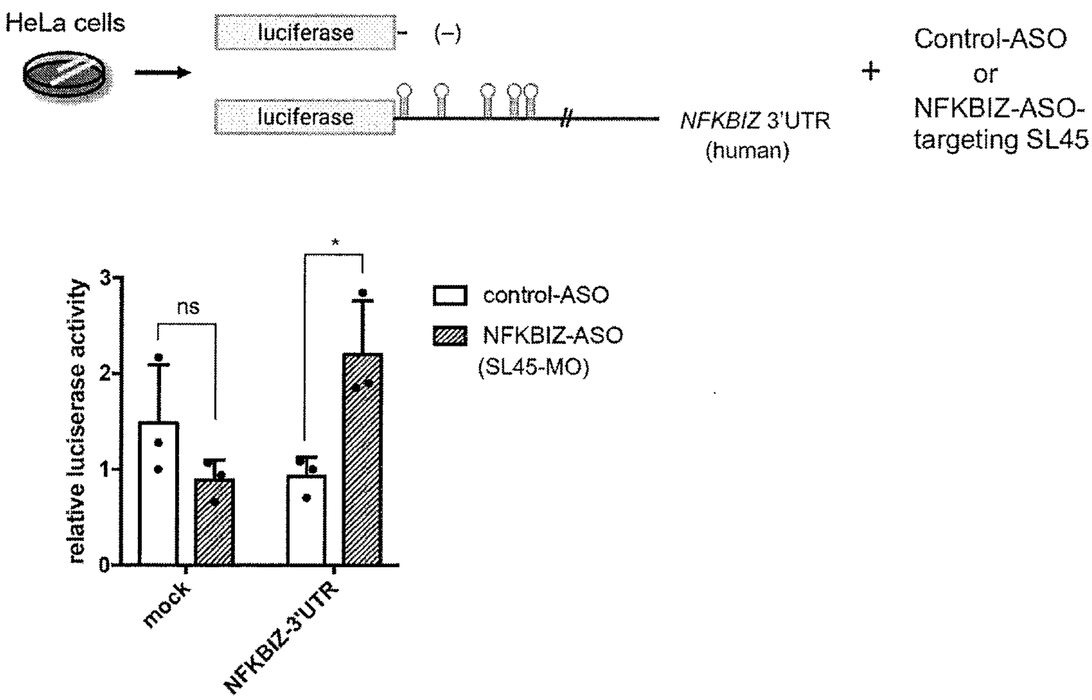
HeLa cells
luciferase
(–)
luciferase
NFKBIZ 3'UTR
(human)
+
Control-ASO
or
NFKBIZ-ASO-
targeting SL45
relative luciserase activity
ns
*
control-ASO
NFKBIZ-ASO
(SL45-MO)
0
1
2
3
mock
NFKBIZ-3'UTR

[Fig. 9]

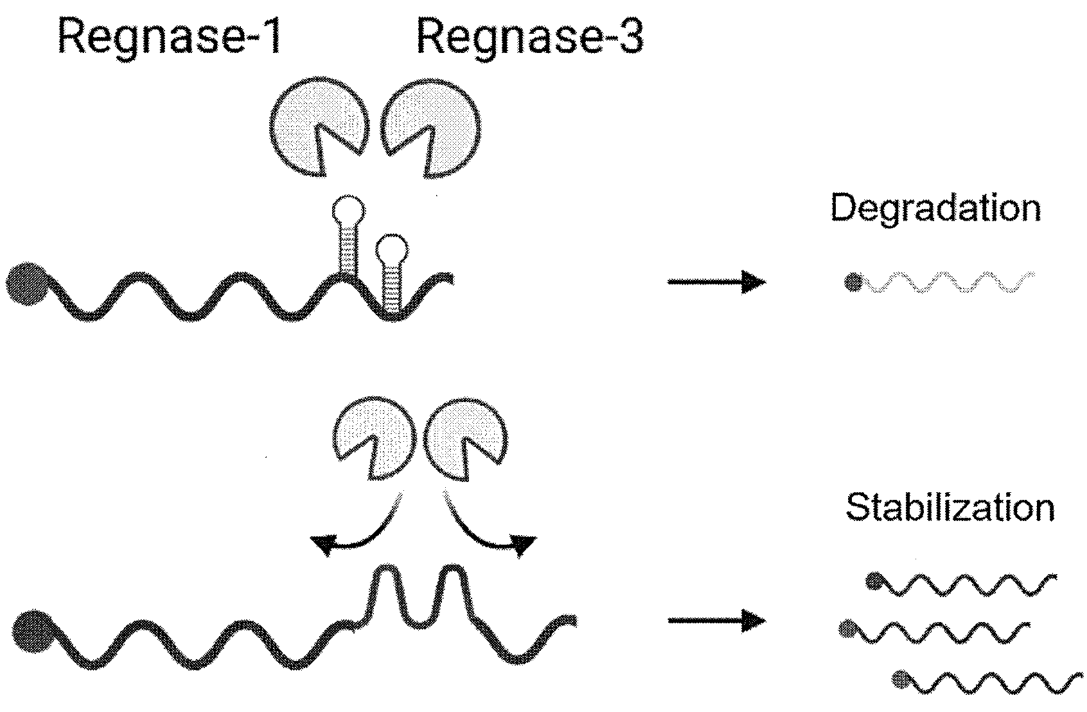
Regnase-1
Regnase-3
Degradation
Stabilization

[Fig. 10]

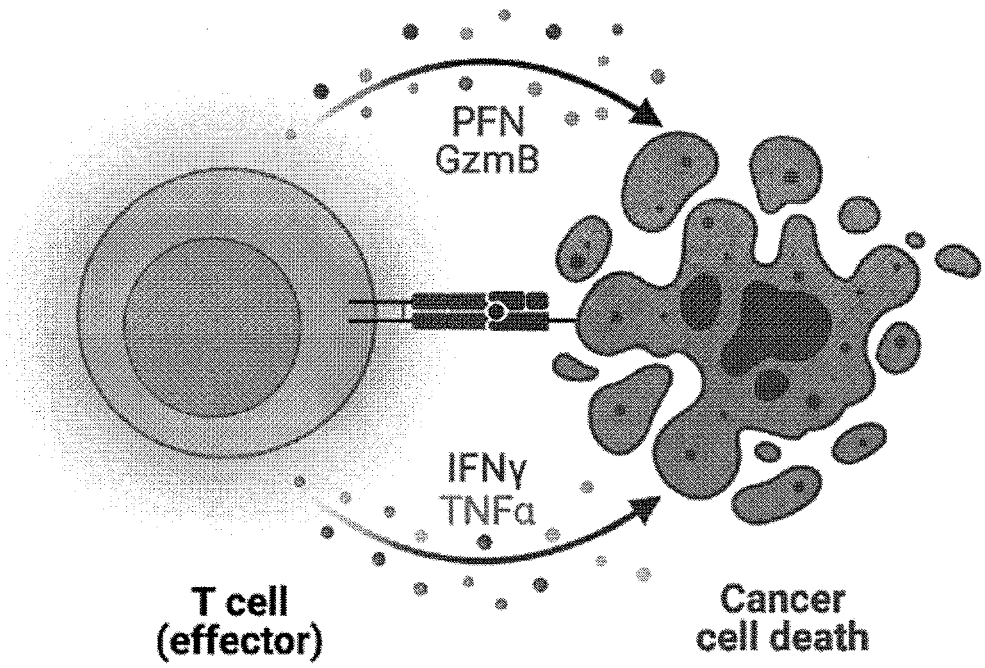
PFN
GzmB
IFNγ
TNFα
T cell
(effector)
Cancer
cell death

[Fig. 11]

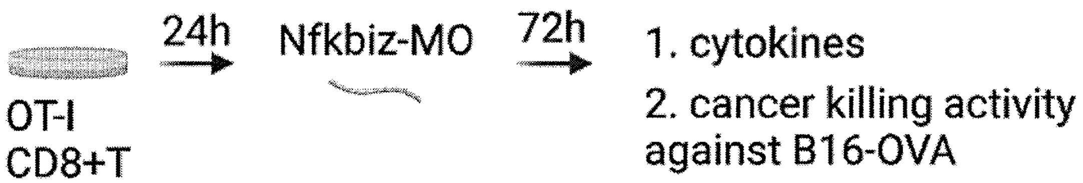
24h
Nfkbiz-MO
72h
1. cytokines
2. cancer killing activity
against B16-OVA
OT-I
CD8+T

[Fig. 12]

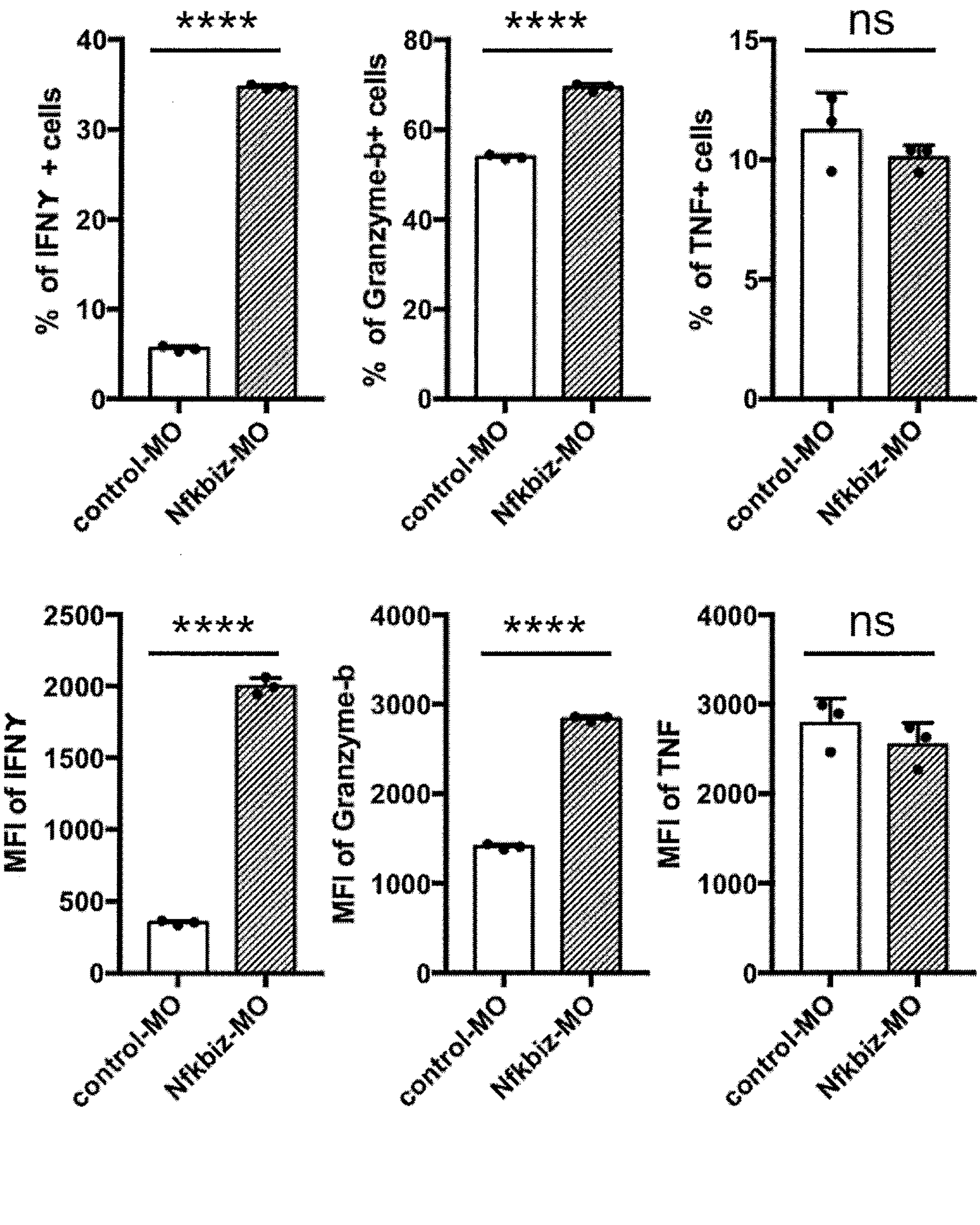
****
% of IFNY + cells
control-MO
Nfkbiz-MO
****
% of Granzyme-b+ cells
ns
% of TNF+ cells
****
MFI of IFNY
****
MFI of Granzyme-b
ns
MFI of TNF

[Fig. 13]

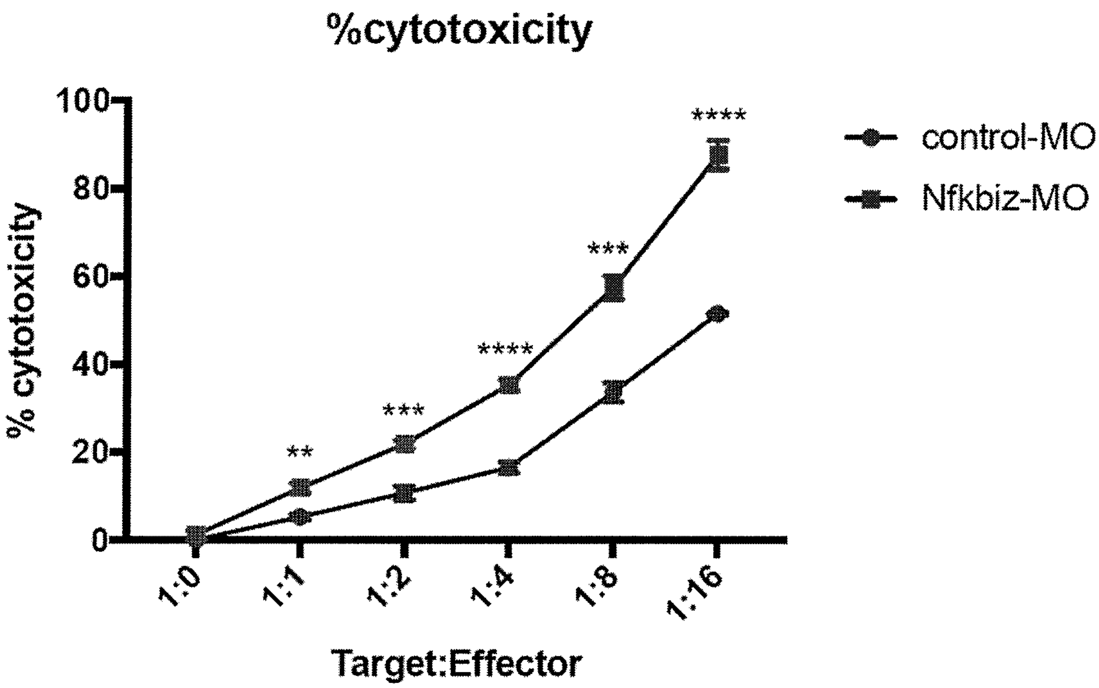
%cytotoxicity
% cytotoxicity
100
80
60
40
20
0
**
***
****
***
****
control-MO
Nfkbiz-MO
1:0
1:1
1:2
1:4
1:8
1:16
Target:Effector

[Fig. 14]

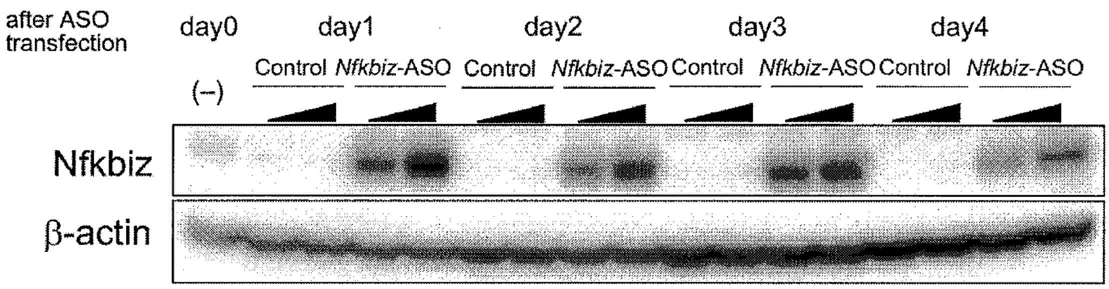
after ASO transfection
day0
day1
day2
day3
day4
Control
Nfkbiz-ASO
(–)
Nfkbiz
β-actin

[Fig. 15]

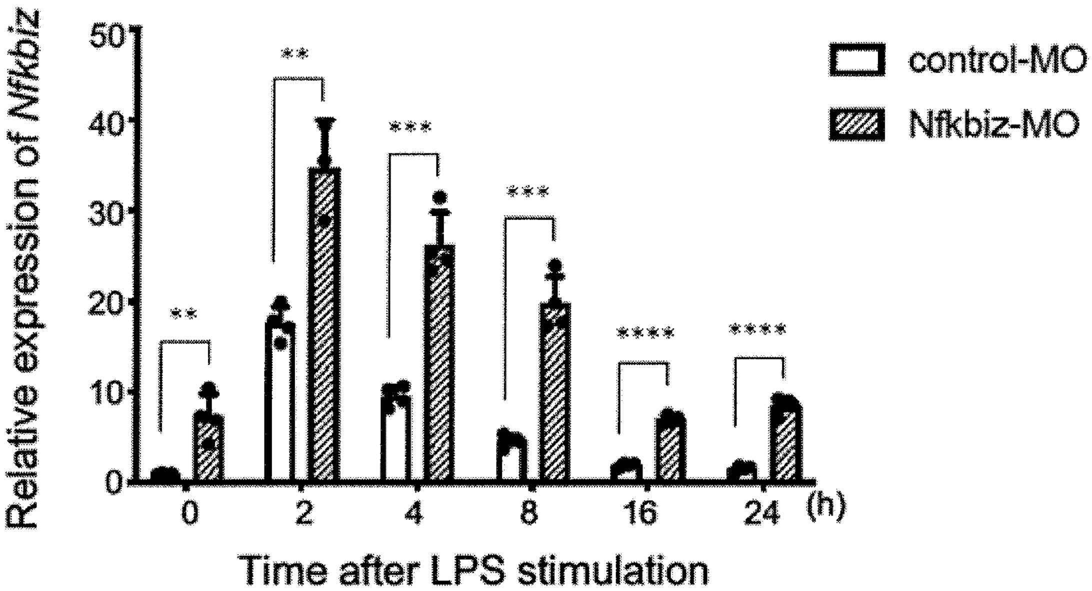
Relative expression of Nfkbiz
50
40
30
20
10
0
**
**
***
***
****
****
0
2
4
8
16
24
(h)
Time after LPS stimulation
control-MO
Nfkbiz-MO

[Fig. 16]

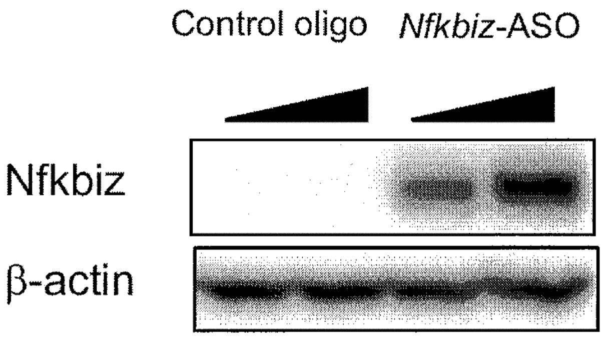
Control oligo
Nfkbiz-ASO
Nfkbiz
β-actin

[Fig. 17]

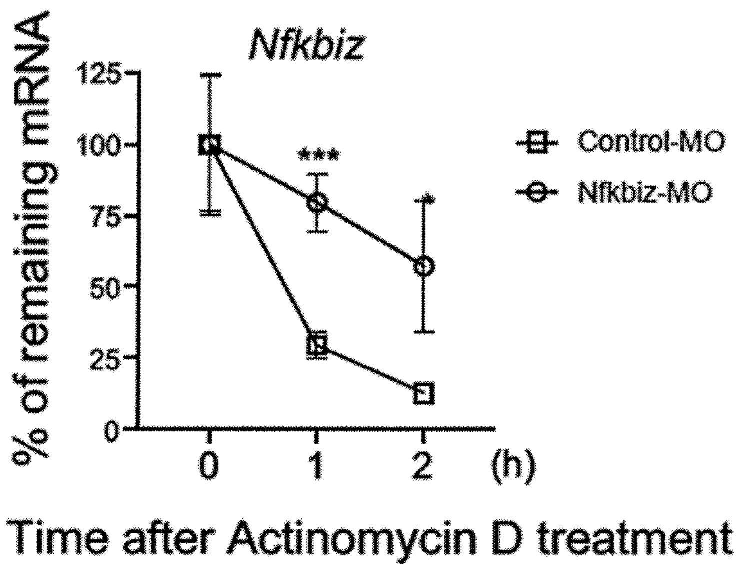
Nfkbiz
% of remaining mRNA
125
100
75
50
25
0
***
*
Control-MO
Nfkbiz-MO
0
1
2
(h)
Time after Actinomycin D treatment

[Fig. 18]

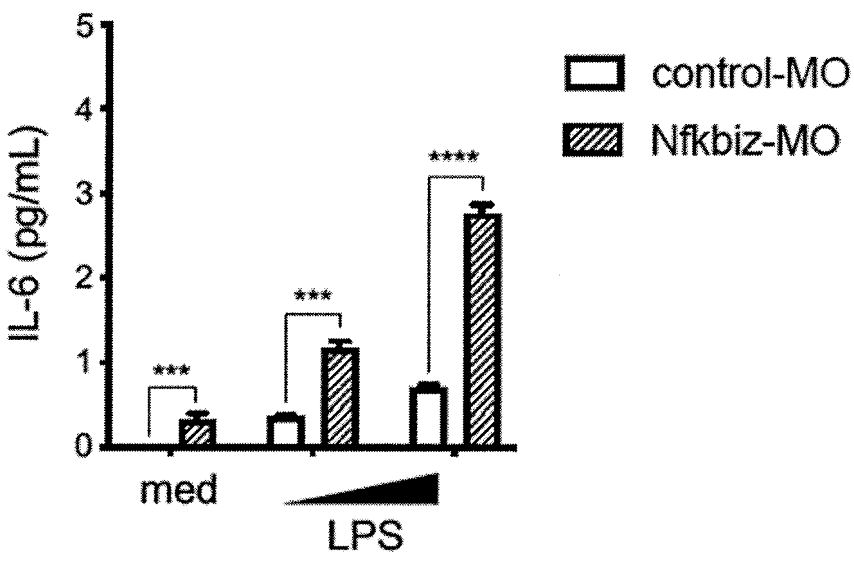
IL-6 (pg/mL)
5
4
3
2
1
0
control-MO
Nfkbiz-MO
***
***
****
med
LPS

[Fig. 19]

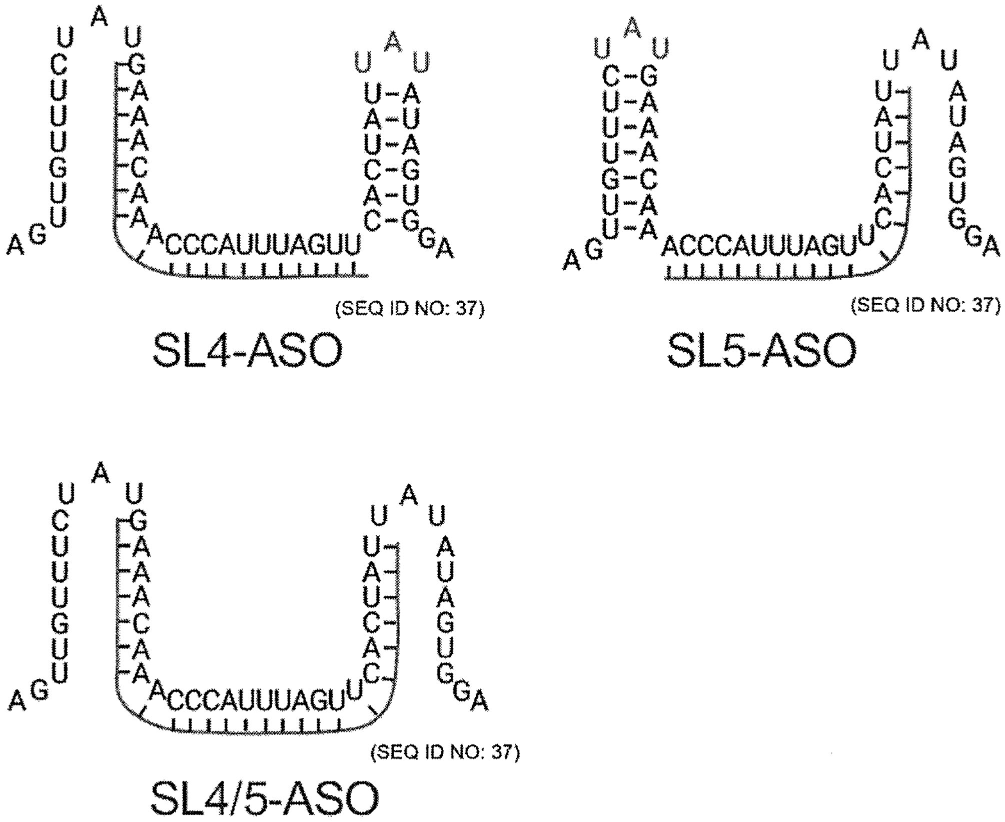
ACCCAUUUAGUU
(SEQ ID NO: 37)
SL4-ASO
ACCCAUUUAGU
(SEQ ID NO: 37)
SL5-ASO
ACCCAUUUAGU
(SEQ ID NO: 37)
SL4/5-ASO

[Fig. 20]

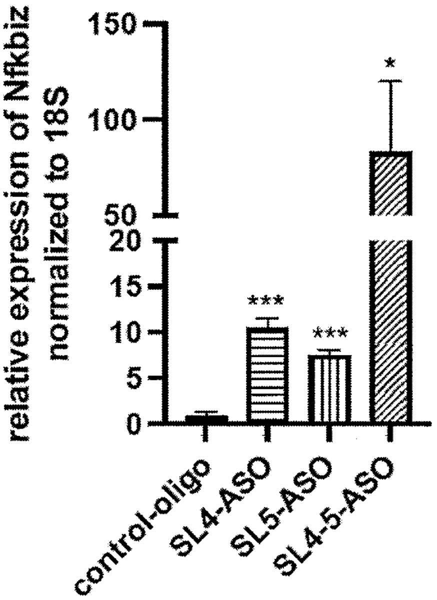
relative expression of Nfkbiz normalized to 18S
150
100
50
20
15
10
5
0
***
***
*
control-oligo
SL4-ASO
SL5-ASO
SL4-5-ASO

[Fig. 21]

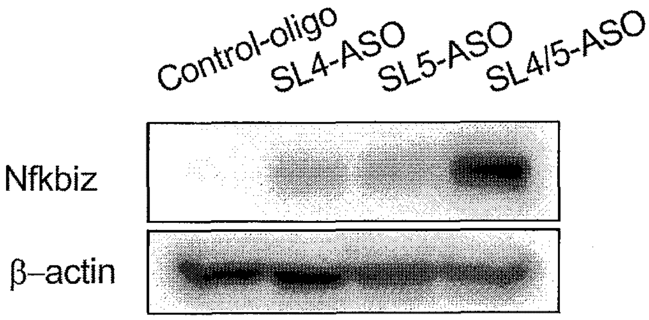


[Fig. 22]

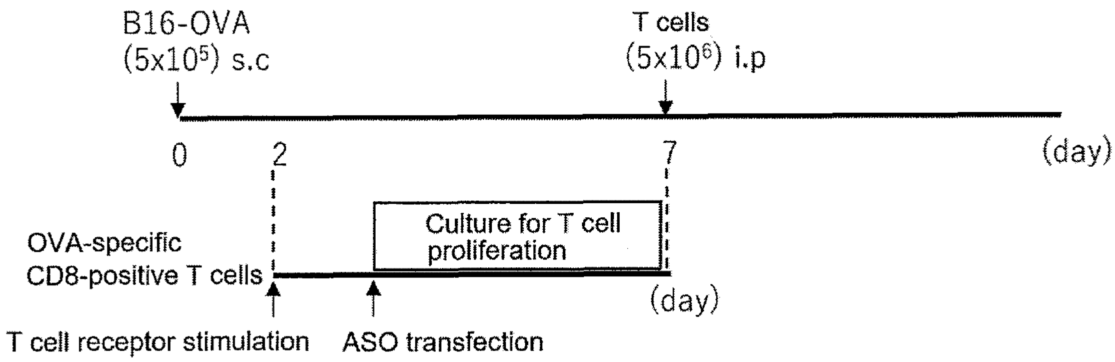


[Fig. 23]

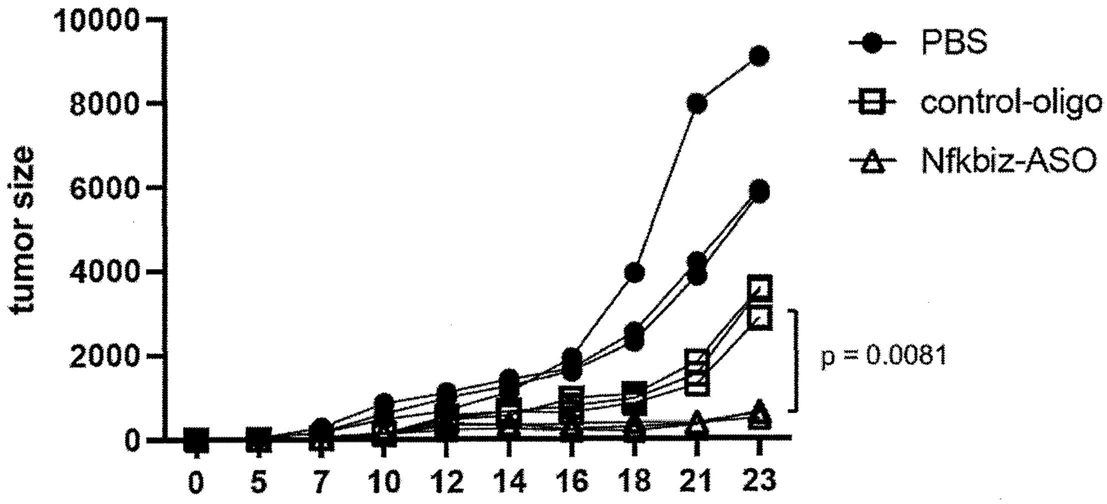

[Fig. 24]

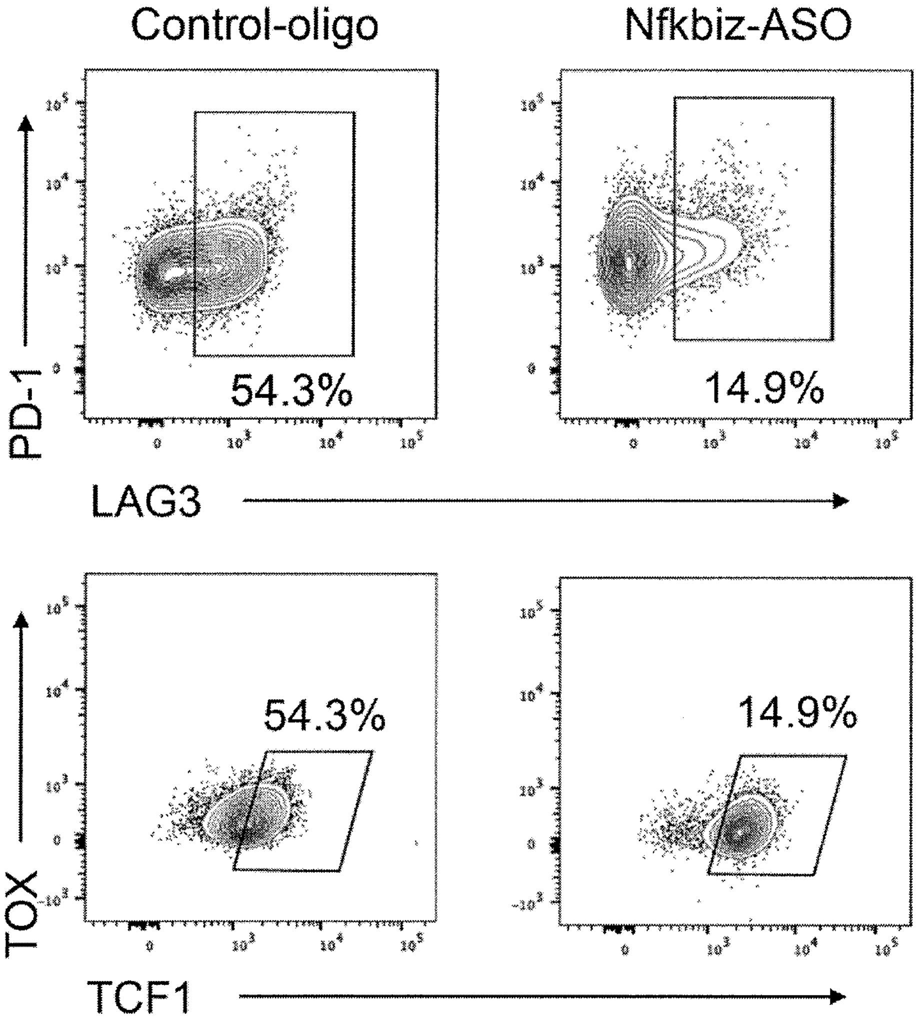
Control-oligo
Nfkbiz-ASO
PD-1
54.3%
14.9%
LAG3
TOX
TCF1
54.3%
14.9%

[Fig. 25]

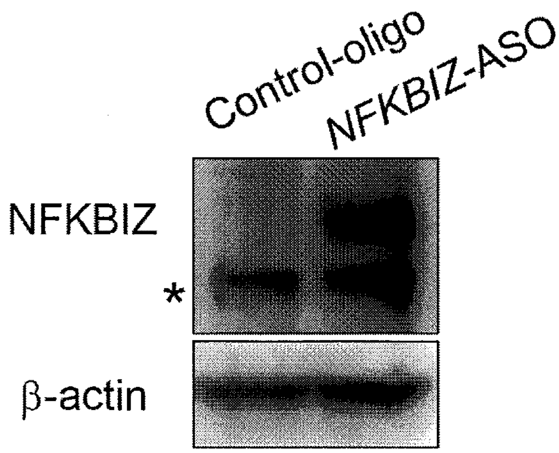
Control-oligo
NFKBIZ-ASO
NFKBIZ
*
β-actin

**INTERNATIONAL SEARCH REPORT**

International application No.
**PCT/JP2023/020104**

**A. CLASSIFICATION OF SUBJECT MATTER**

***C12N 15/11***(2006.01)i; ***A61K 31/7088***(2006.01)i; ***A61K 45/00***(2006.01)i; ***A61K 48/00***(2006.01)i; ***A61P 35/00***(2006.01)i; ***A61P 37/04***(2006.01)i; ***A61P 43/00***(2006.01)i
FI: C12N15/11 Z ZNA; A61P43/00 121; A61P37/04; A61P35/00; A61K45/00; A61K48/00; A61K31/7088

According to International Patent Classification (IPC) or to both national classification and IPC

**B. FIELDS SEARCHED**

Minimum documentation searched (classification system followed by classification symbols)

C12N15/11; A61K31/7088; A61K45/00; A61K48/00; A61P35/00; A61P37/04; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | BEHRENS, G. et al. A translational silencing function of MCPIP1/Regnase-1 specified by the target site context. Nucleic Acids Research. 2018, vol. 46, no. 8, pp. 4256-4270<br>entirety in particular, abstract, p. 4257, left column, p. 4261, fig. 2, p. 4263, fig. 4 | 1-11 |
| Y | TSE, K. M. et al. Enhancement of Regnase-1 expression with stem loop-targeting antisense oligonucleotides alleviates inflammatory diseases. Science Translational Medicine. 11 May 2022, vol. 14, eabo2137, pp. 1-14<br>entirety in particular, abstract, p. 3, fig. 1 | 1-11 |
| Y | XU, T. et al. Upregulation of NFKBIZ affects bladder cancer progression via the PTEN/PI3K/Akt signaling pathway. International Journal of Molecular Medicine. 2021, vol. 47, no. 109, pp. 1-12<br>entirety in particular, abstract, pp. 5-7 | 1-11 |
| Y | MIYAKE, T. et al. IκBζ is essential for natural killer cell activation in response to IL-12 and IL-18. Proceedings of the National Academy of Sciences of the United States of America. 2010, vol. 107, no. 41, pp. 17680-17685<br>entirety in particular, abstract, p. 17683 | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 August 2023** | **15 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2023/020104**

**C. DOCUMENTS CONSIDERED TO BE RELEVANT**

| Category* | Citation of document, with indication, where appropriate, of the relevant passages | Relevant to claim No. |
|---|---|---|
| A | WO 2021/025018 A1 (UNIV KYOTO) 11 February 2021 (2021-02-11) entirety in particular, abstract, claims, examples | 1-11 |

Form PCT/ISA/210 (second sheet) (January 2015)

**INTERNATIONAL SEARCH REPORT**

International application No.
**PCT/JP2023/020104**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

"The form of Annex C/ST.25 text file" above shall read as "the form of ST.26.".

Form PCT/ISA/210 (continuation of first sheet) (January 2015)

**INTERNATIONAL SEARCH REPORT**
**Information on patent family members**

International application No.
**PCT/JP2023/020104**

| Patent document cited in search report | Publication date (day/month/year) | Patent family member(s) | Publication date (day/month/year) |
|---|---|---|---|
| WO 2021/025018 A1 | 11 February 2021 | JP 2022-137317 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022088931 A
- WO 2019182055 A


### Non-patent literature cited in the description


- **MINO, T. et al.** *Cell*, 2015, vol. 161, 1058-1073
- **VON GAMM M. et al.** *J Exp Med*, 2019, vol. 216 (7), 1700-1723
- **DELEAVEY, G. F. ; DAMHA, M. J.** Designing chemically modified oligonucleotides for targeted gene silencing. *Chemistry & biology*, 2012, vol. 19 (8), 937-954
- Molecular Cloning: A Laboratory Manual. Cold Spring Harbor Laboratory Press, 1989
- **UEHATA, T. et al.** *Cell*, 2013, vol. 153, 1036-1049
- **OKUMA A. et al.** *Immunity*, 21 March 2013, vol. 38 (3), 450-60
- **VENTURA A. et al.** *Nature*, 08 February 2007, vol. 445 (7128), 661-5
- **HOGQUIST K.A. et al.** *Cell*, 14 January 1994, vol. 76 (1), 17-27